# EUROPEAN PATENT APPLICATION

(11) **EP 4 332 223 A1**
(43) Date of publication of application: **06.03.2024**
(21) Application number: 22795887.3
(22) Date of filing: 28.04.2022
(51) Int. Cl.: C12N 15/113, A61K 31/7088, A61K 31/712, A61K 31/7125, A61K 31/713, A61K 48/00, A61P 31/12, C12N 15/63

(54) **ANTIVIRAL NUCLEIC ACID**

(30) Priority: 28.04.2021 JP 2021076521
(71) Applicant: Nippon Shinyaku Co., Ltd., Kyoto-shi Kyoto 601-8550 (JP); National University Corporation Gunma University, Maebashi-shi, Gunma 371-8510 (JP)
(72) Inventor: NAKAGAWA Shinichiro, Tsukuba-shi, Ibaraki 305-0003 (JP); TAGAYA Mitsuhiro, Tsukuba-shi, Ibaraki 305-0003 (JP); HIMOTO Takuya, Tsukuba-shi, Ibaraki 305-0003 (JP); KAMITANI Wataru, Maebashi-shi, Gunma 371-8510 (JP)
(74) Representative: Hoffmann Eitle
(86) International application number: PCT/JP2022/019273
(87) International publication number: WO 2022/230974

(57) **Abstract**

An object of the present invention is to provide an antiviral nucleic acid, etc. against SARS-CoV-2 SARS-CoV-2, SARS-CoV-1, or MERS-CoV and/or a method for treating and/or preventing viral infection using the nucleic acid, etc. The present invention relates to, for example, an antiviral nucleic acid or a pharmaceutically acceptable salt or hydrate thereof targeting a sequence in at least one target region selected from the group consisting of 5' UTR region, nsp3 region, 3C-like proteinase region, nsp9 region, RNA-dependent RNA polymerase region, helicase region, 3'-to-5' exonuclease region, 2'-O-ribose methyltransferase region, S region including S1 region and S2 region, E region, M region, and N region in genomic RNA of SARS-CoV-2, wherein the virus is SARS-CoV-2, SARS-CoV-1, or MERS-CoV.

## Description

### Technical Field

The present invention relates to an antiviral nucleic acid or a pharmaceutically acceptable salt or hydrate thereof against SARS-CoV-2, SARS-CoV-1, or MERS-CoV (hereinafter, also referred to as the " nucleic acid, etc."); a vector comprising the nucleic acid, etc.; a pharmaceutical composition comprising the nucleic acid, etc. or the vector; or a method for treating and/or preventing viral infection, comprising administering to a subject the nucleic acid, etc., the vector, or the pharmaceutical composition.

### Background Art

COVID-19 (coronavirus disease-2019) is a novel infection characterized by pneumonia which was first confirmed in Wuhan, Hubei Province, China in November 2019 and then declared a pandemic by WHO in March 2020 (Non Patent Literature 1). The cause of this COVID-19 is a novel virus, and the causative virus was identified as severe acute respiratory syndrome coronavirus 2 (SARS-CoV-2) in January 2020. SARS-CoV-2 is evolutionarily closely related to SARS-CoV, a causative virus of severe acute respiratory syndrome (SARS) spread in 2003, and belongs to the genus *Betacoronavirus,* as in SARS-CoV (Non Patent Literature 2).

The viral genome of SARS-CoV-2 is constituted by single-stranded plus-strand RNA of about 30,000 bases. Thus, the viral genome of SARS-CoV-2 is considered capable of serving as a substrate for cleavage by RNA interference, and siRNA that exerts knockdown activity against the viral genome of SARS-CoV-2 can be expected to be applied to a therapeutic agent for COVID-19 as an anti-SARS-CoV-2 agent. Sequence information on some siRNAs against the genome of SARS-CoV-2 has been disclosed so far (Patent Literatures 1 and 2).

### Citation List

### Patent Literature

Patent Literature 1: CN111139241A
Patent Literature 2: CN111139242A

### Non Patent Literature

Non Patent Literature 1: World Health Organization (WHO) (Press release). 11 March 2020
Non Patent Literature 2: Nat Microbiol. 2020 Apr; 5 (4): 536-544

### Summary of Invention

Although use of existing antiviral agents, for example, has been proposed for SARS-CoV-2, their effects have not yet been clinically demonstrated and there is no established treatment method. Also, there is no established treatment method as to SARS-CoV-1 and MERS-CoV which belong to the genus *Betacoronavirus* and cause viral infection, as in SARS-CoV-2

An object of the present invention is to provide an antiviral nucleic acid, etc. against SARS-CoV-2, SARS-CoV-1, or MERS-CoV, and/or a method for treating and/or preventing viral infection using the nucleic acid, etc.

The present inventors have accomplished the present invention by finding that an antiviral nucleic acid or a pharmaceutically acceptable salt or hydrate thereof targeting a sequence in at least one target region selected from the group consisting of 5' UTR region, nsp3 region, 3C-like proteinase region, nsp9 region, RNA-dependent RNA polymerase region, helicase region, 3'-to-5' exonuclease region, 2'-O-ribose methyltransferase region, S region including S1 region and S2 region, E region, M region, and N region in genomic RNA of SARS-CoV-2 is capable of exerting an antiviral effect on SARS-CoV-2, SARS-CoV-1, or MERS-CoV.

The present invention encompasses the following embodiments.
(1) An antiviral nucleic acid or a pharmaceutically acceptable salt or hydrate thereof targeting a sequence in at least one target region selected from the group consisting of 5' UTR region, nsp3 region, 3C-like proteinase region, nsp9 region, RNA-dependent RNA polymerase region, helicase region, 3'-to-5' exonuclease region, 2'-O-ribose methyltransferase region, S region including S1 region and S2 region, E region, M region, and N region in genomic RNA of SARS-CoV-2, wherein
   the virus is SARS-CoV-2, SARS-CoV-1, or MERS-CoV.
(2) The nucleic acid or the pharmaceutically acceptable salt or hydrate thereof according to (1), wherein the target sequence is a sequence of 16 to 30 consecutive bases in the target region.
(3) The nucleic acid or the pharmaceutically acceptable salt or hydrate thereof according to (1) or (2), wherein the virus is SARS-CoV-2 or SARS-CoV-1.
(4-1) The nucleic acid or the pharmaceutically acceptable salt or hydrate thereof according to (3), wherein the nucleic acid or the pharmaceutically acceptable salt or hydrate thereof targets a sequence in at least one target region selected from the group consisting of positions 43 to 69, positions 242 to 279, positions 3352 to 3372, positions 6406 to 6427, positions 10406 to 10431, positions 10484 to 10506, positions 12839 to 12867, positions 12898 to 12922, positions 12965 to 12990, positions 13481 to 13502, positions 13762 to 13790, positions 13894 to 13916, positions 14290 to 14312, positions 14654 to 14687, positions 14750 to 14777, positions 14824 to 14846, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15622 to 15644, positions 15829 to 15859, positions 15929 to 15950, positions 15985 to 16011, positions 16051 to 16085, positions 16189 to 16220, positions 16430 to 16451, positions 16822 to 16845, positions 17015 to 17051, positions 17080 to 17103, positions 17254 to 17277, positions 17564 to 17600, positions 18196 to 18218, positions 18253 to 18278, positions 19568 to 19595, positions 20888 to 20909, positions 21502 to 21524, positions 22814 to 22836, positions 23093 to 23113, positions 23956 to 23976, positions 24302 to 24324, positions 24467 to 24489, positions 24620 to 24651, positions 24662 to 24684, positions 24962 to 24982, positions 25104 to 25128, positions 25364 to 25384, positions 26287 to 26325, positions 26574 to 26604, positions 28274 to 28294, positions 28397 to 28418, positions 28509 to 28538, positions 28744 to 28784, positions 28799 to 28820, positions 28946 to 28972, positions 29010 to 29031, positions 29102 to 29130, and positions 29174 to 29196 of SEQ ID NO: 1.
(4-2) The nucleic acid or the pharmaceutically acceptable salt or hydrate thereof according to (4-1), wherein the nucleic acid or the pharmaceutically acceptable salt or hydrate thereof targets a sequence in at least one target region selected from the group consisting of positions 43 to 63, positions 44 to 64, positions 45 to 65, positions 46 to 66, positions 47 to 67, positions 48 to 68, positions 49 to 69, positions 242 to 262, positions 243 to 263, positions 244 to 264, positions 245 to 265, positions 246 to 266, positions 247 to 267, positions 248 to 268, positions 249 to 269, positions 250 to 270, positions 251 to 271, positions 252 to 272, positions 253 to 273, positions 254 to 274, positions 255 to 275, positions 256 to 276, positions 257 to 277, positions 258 to 278, positions 259 to 279, positions 3352 to 3372, positions 6406 to 6426, positions 6407 to 6427, positions 10406 to 10426, positions 10407 to 10427, positions 10408 to 10428, positions 10409 to 10429, positions 10410 to 10430, positions 10411 to 10431, positions 10484 to 10504, positions 10485 to 10505, positions 10486 to 10506, positions 12839 to 12859, positions 12840 to 12860, positions 12841 to 12861, positions 12842 to 12862, positions 12843 to 12863, positions 12844 to 12864, positions 12845 to 12865, positions 12846 to 12866, positions 12847 to 12867, positions 12898 to 12918, positions 12899 to 12919, positions 12900 to 12920, positions 12901 to 12921, positions 12902 to 12922, positions 12965 to 12985, positions 12966 to 12986, positions 12967 to 12987, positions 12968 to 12988, positions 12969 to 12989, positions 12970 to 12990, positions 13481 to 13501, positions 13482 to 13502, positions 13762 to 13782, positions 13763 to 13783, positions 13764 to 13784, positions 13765 to 13785, positions 13766 to 13786, positions 13767 to 13787, positions 13768 to 13788, positions 13769 to 13789, positions 13770 to 13790, positions 13894 to 13914, positions 13895 to 13915, positions 13896 to 13916, positions 14290 to 14310, positions 14291 to 14311, positions 14292 to 14312, positions 14654 to 14674, positions 14655 to 14675, positions 14656 to 14676, positions 14657 to 14677, positions 14658 to 14678, positions 14659 to 14679, positions 14660 to 14680, positions 14661 to 14681, positions 14662 to 14682, positions 14663 to 14683, positions 14664 to 14684, positions 14665 to 14685, positions 14666 to 14686, positions 14667 to 14687, positions 14750 to 14770, positions 14751 to 14771, positions 14752 to 14772, positions 14753 to 14773, positions 14754 to 14774, positions 14755 to 14775, positions 14756 to 14776, positions 14757 to 14777, positions 14824 to 14844, positions 14825 to 14845, positions 14826 to 14846, positions 14878 to 14898, positions 14879 to 14899, positions 14880 to 14900, positions 14881 to 14901, positions 14882 to 14902, positions 14883 to 14903, positions 14884 to 14904, positions 14885 to 14905, positions 14886 to 14906, positions 14887 to 14907, positions 14888 to 14908, positions 14889 to 14909, positions 14953 to 14973, positions 14954 to 14974, positions 14955 to 14975, positions 14956 to 14976, positions 14957 to 14977, positions 14958 to 14978, positions 14959 to 14979, positions 14960 to 14980, positions 14961 to 14981, positions 14962 to 14982, positions 14963 to 14983, positions 14964 to 14984, positions 14965 to 14985, positions 14966 to 14986, positions 14967 to 14987, positions 14968 to 14988, positions 14969 to 14989, positions 14970 to 14990, positions 14992 to 15012, positions 14993 to 15013, positions 14994 to 15014, positions 14995 to 15015, positions 14996 to 15016, positions 14997 to 15017, positions 14998 to 15018, positions 14999 to 15019, positions 15000 to 15020, positions 15001 to 15021, positions 15002 to 15022, positions 15003 to 15023, positions 15004 to 15024, positions 15005 to 15025, positions 15006 to 15026, positions 15037 to 15057, positions 15038 to 15058, positions 15039 to 15059, positions 15040 to 15060, positions 15041 to 15061, positions 15063 to 15083, positions 15064 to 15084, positions 15065 to 15085, positions 15066 to 15086, positions 15067 to 15087, positions 15068 to 15088, positions 15069 to 15089, positions 15070 to 15090, positions 15071 to 15091, positions 15072 to 15092, positions 15073 to 15093, positions 15074 to 15094, positions 15075 to 15095, positions 15076 to 15096, positions 15077 to 15097, positions 15078 to 15098, positions 15079 to 15099, positions 15080 to 15100, positions 15081 to 15101, positions 15082 to 15102, positions 15083 to 15103, positions 15084 to 15104, positions 15085 to 15105, positions 15086 to 15106, positions 15087 to 15107, positions 15088 to 15108, positions 15089 to 15109, positions 15090 to 15110, positions 15091 to 15111, positions 15092 to 15112, positions 15093 to 15113, positions 15094 to 15114, positions 15095 to 15115, positions 15096 to 15116, positions 15097 to 15117, positions 15098 to 15118, positions 15099 to 15119, positions 15100 to 15120, positions 15101 to 15121, positions 15102 to 15122, positions 15103 to 15123, positions 15104 to 15124, positions 15105 to 15125, positions 15106 to 15126, positions 15107 to 15127, positions 15108 to 15128, positions 15109 to 15129, positions 15110 to 15130, positions 15111 to 15131, positions 15112 to 15132, positions 15113 to 15133, positions 15114 to 15134, positions 15115 to 15135, positions 15116 to 15136, positions 15117 to 15137, positions 15118 to 15138, positions 15119 to 15139, positions 15120 to 15140, positions 15172 to 15192, positions 15173 to 15193, positions 15174 to 15194, positions 15175 to 15195, positions 15176 to 15196, positions 15177 to 15197, positions 15178 to 15198, positions 15278 to 15298, positions 15279 to 15299, positions 15454 to 15474, positions 15455 to 15475, positions 15456 to 15476, positions 15457 to 15477, positions 15458 to 15478, positions 15459 to 15479, positions 15496 to 15516, positions 15497 to 15517, positions 15498 to 15518, positions 15622 to 15642, positions 15623 to 15643, positions 15624 to 15644, positions 15829 to 15849, positions 15830 to 15850, positions 15831 to 15851, positions 15832 to 15852, positions 15833 to 15853, positions 15834 to 15854, positions 15835 to 15855, positions 15836 to 15856, positions 15837 to 15857, positions 15838 to 15858, positions 15839 to 15859, positions 15929 to 15949, positions 15930 to 15950, positions 15985 to 16005, positions 15986 to 16006, positions 15987 to 16007, positions 15988 to 16008, positions 15989 to 16009, positions 15990 to 16010, positions 15991 to 16011, positions 16051 to 16071, positions 16052 to 16072, positions 16053 to 16073, positions 16054 to 16074, positions 16055 to 16075, positions 16056 to 16076, positions 16057 to 16077, positions 16058 to 16078, positions 16059 to 16079, positions 16060 to 16080, positions 16061 to 16081, positions 16062 to 16082, positions 16063 to 16083, positions 16064 to 16084, positions 16065 to 16085, positions 16189 to 16209, positions 16190 to 16210, positions 16191 to 16211, positions 16192 to 16212, positions 16193 to 16213, positions 16194 to 16214, positions 16195 to 16215, positions 16196 to 16216, positions 16197 to 16217, positions 16198 to 16218, positions 16199 to 16219, positions 16200 to 16220, positions 16430 to 16450, positions 16431 to 16451, positions 16822 to 16842, positions 16823 to 16842, positions 16823 to 16843, positions 16824 to 16844, positions 16825 to 16845, positions 17015 to 17035, positions 17016 to 17036, positions 17017 to 17037, positions 17018 to 17038, positions 17019 to 17039, positions 17020 to 17040, positions 17021 to 17041, positions 17022 to 17042, positions 17023 to 17043, positions 17024 to 17044, positions 17025 to 17045, positions 17026 to 17046, positions 17027 to 17047, positions 17028 to 17048, positions 17029 to 17049, positions 17030 to 17050, positions 17031 to 17051, positions 17080 to 17100, positions 17081 to 17101, positions 17082 to 17102, positions 17083 to 17103, positions 17254 to 17274, positions 17255 to 17275, positions 17256 to 17276, positions 17257 to 17277, positions 17564 to 17584, positions 17565 to 17585, positions 17566 to 17586, positions 17567 to 17587, positions 17568 to 17588, positions 17569 to 17589, positions 17570 to 17590, positions 17571 to 17591, positions 17572 to 17592, positions 17573 to 17593, positions 17574 to 17594, positions 17575 to 17595, positions 17576 to 17596, positions 17577 to 17597, positions 17578 to 17598, positions 17579 to 17599, positions 17580 to 17600, positions 18196 to 18216, positions 18197 to 18217, positions 18198 to 18218, positions 18253 to 18273, positions 18254 to 18274, positions 18255 to 18275, positions 18256 to 18276, positions 18257 to 18277, positions 18258 to 18278, positions 19568 to 19588, positions 19569 to 19589, positions 19570 to 19590, positions 19571 to 19591, positions 19572 to 19592, positions 19573 to 19593, positions 19574 to 19594, positions 19575 to 19595, positions 20888 to 20908, positions 20889 to 20909, positions 21502 to 21522, positions 21503 to 21523, positions 21504 to 21524, positions 22814 to 22834, positions 22815 to 22835, positions 22816 to 22836, positions 23093 to 23113, positions 23956 to 23976, positions 24302 to 24322, positions 24303 to 24333, positions 24304 to 24324, positions 24467 to 24487, positions 24468 to 24488, positions 24469 to 24489, positions 24620 to 24640, positions 24621 to 24641, positions 24622 to 24642, positions 24623 to 24643, positions 24624 to 24644, positions 24625 to 24645, positions 24626 to 24646, positions 24627 to 24647, positions 24628 to 24648, positions 24629 to 24649, positions 24630 to 24650, positions 24631 to 24651, positions 24662 to 24682, positions 24663 to 24683, positions 24664 to 24684, positions 24962 to 24982, positions 25104 to 25124, positions 25105 to 25125, positions 25106 to 25126, positions 25107 to 25127, positions 25108 to 25128, positions 25364 to 25384, positions 26287 to 26307, positions 26288 to 26308, positions 26289 to 26309, positions 26290 to 26310, positions 26291 to 26311, positions 26292 to 26312, positions 26293 to 26313, positions 26294 to 26314, positions 26295 to 26315, positions 26296 to 26316, positions 26297 to 26317, positions 26298 to 26318, positions 26299 to 26319, positions 26300 to 26320, positions 26301 to 26321, positions 26302 to 26322, positions 26303 to 26323, positions 26304 to 26324, positions 26305 to 26325, positions 26574 to 26594, positions 26575 to 26595, positions 29576 to 29596, positions 26577 to 26597, positions 26578 to 26598, positions 26579 to 26599, positions 26580 to 26600, positions 26581 to 26601, positions 26582 to 26602, positions 26583 to 26603, positions 26584 to 26604, positions 28273 to 28293, positions 28274 to 28294, positions 28397 to 28417, positions 28398 to 28418, positions 28509 to 28529, positions 28510 to 28530, positions 28511 to 28531, positions 28512 to 28532, positions 28513 to 28533, positions 28514 to 28534, positions 28515 to 28535, positions 28516 to 28536, positions 28517 to 28537, positions 28518 to 28538, positions 28744 to 288764, positions 28745 to 28765, positions 28746 to 28766, positions 28747 to 28767, positions 28748 to 28768, positions 28749 to 28769, positions 28750 to 28770, positions 28751 to 28771, positions 28752 to 28772, positions 28753 to 28773, positions 28754 to 28774, positions 28755 to 28775, positions 28756 to 28776, positions 28757 to 28777, positions 28758 to 28778, positions 28759 to 28779, positions 28760 to 28780, positions 28761 to 28781, positions 28762 to 28782, positions 28763 to 28783, positions 28764 to 28784, positions 28799 to 28819, positions 28800 to 28820, positions 28946 to 28966, positions 28947 to 28967, positions 28948 to 28968, positions 28949 to 28969, positions 28950 to 28970, positions 28951 to 28971, positions 28952 to 28972, positions 29010 to 29030, positions 29011 to 29031, positions 29102 to 29122, positions 29103 to 29123, positions 29104 to 29124, positions 29105 to 29125, positions 29106 to 29126, positions 29107 to 29127, positions 29108 to 29128, positions 29109 to 29129, positions 29110 to 29130, positions 29174 to 29194, position 29175, and positions 29176 to 29196 of SEQ ID NO: 1.
(4-3) The nucleic acid or the pharmaceutically acceptable salt or hydrate thereof according to (4-1), wherein the nucleic acid or the pharmaceutically acceptable salt or hydrate thereof targets a sequence in at least one target region selected from the group consisting of positions 43 to 63, positions 48 to 68, positions 3352 to 3372, positions 6406 to 6426, positions 10410 to 10430, positions 10411 to 10431, positions 10486 to 10506, positions 12898 to 12918, positions 13763 to 13783, positions 13765 to 13785, positions 13895 to 13915, positions 14883 to 14903, positions 14887 to 14907, positions 14955 to 15015, positions 15002 to 15022, positions 15077 to 15097, positions 15079 to 15099, positions 15105 to 15125, positions 15107 to 15127, positions 15110 to 15130, positions 15113 to 15133, positions 15116 to 15136, positions 15498 to 15518, positions 15623 to 15643, positions 15838 to 15858, positions 15987 to 16007, positions 16061 to 16081, positions 16063 to 16083, positions 16193 to 16213, positions 16430 to 16450, positions 16825 to 16845, positions 17022 to 17042, positions 17025 to 17045, positions 17026 to 17046, positions 17081 to 17101, positions 17255 to 17275, positions 17257 to 17277, positions 17565 to 17585, positions 17567 to 17587, positions 19570 to 19590, positions 21504 to 21524, positions 22815 to 22835, positions 22816 to 22836, positions 23956 to 23976, positions 24303 to 24323, positions 24624 to 24644, positions 24625 to 24645, positions 24630 to 24650, positions 24664 to 24684, positions 25364 to 25384, positions 26291 to 26311, positions 26303 to 26323, positions 26579 to 26599, positions 26583 to 26603, positions 26584 to 26604, positions 28746 to 28766, positions 28749 to 28769, positions 28752 to 28772, and positions 28952 to 28972 of SEQ ID NO: 1.
(4-4) The nucleic acid or the pharmaceutically acceptable salt or hydrate thereof according to (4-1), wherein the nucleic acid or the pharmaceutically acceptable salt or hydrate thereof targets a sequence in at least one target region selected from the group consisting of positions 43 to 63, positions 48 to 68, positions 3352 to 3372, positions 6406 to 6426, positions 10486 to 10506, positions 12898 to 12918, positions 13765 to 13785, positions 13895 to 13915, positions 14883 to 14903, positions 14887 to 14907, positions 14955 to 15015, positions 15002 to 15022, positions 15077 to 15097, positions 15079 to 15099, positions 15105 to 15125, positions 16061 to 16081, positions 16063 to 16083, positions 17025 to 17045, positions 17026 to 17046, positions 17081 to 17101, positions 17255 to 17275, positions 17565 to 17585, positions 17567 to 17587, positions 21504 to 21524, positions 24630 to 24650, positions 26583 to 26603, positions 28749 to 28769, positions 28752 to 28772, and positions 28952 to 28972 of SEQ ID NO: 1.
(5-1) The nucleic acid or the pharmaceutically acceptable salt or hydrate thereof according to (1), wherein the nucleic acid or the pharmaceutically acceptable salt or hydrate thereof targets a sequence in at least one target region selected from the group consisting of 5' UTR region, helicase region, and N region in genomic RNA of SARS-CoV-2.
(5-2) The nucleic acid or the pharmaceutically acceptable salt or hydrate thereof according to (5-1), wherein the nucleic acid or the pharmaceutically acceptable salt or hydrate thereof targets a sequence in at least one target region selected from the group consisting of positions 43 to 69, positions 16430 to 16451, positions 16822 to 16845, positions 17015 to 17051, positions 17080 to 17103, positions 17254 to 17277, positions 17564 to 17600, positions 28274 to 28294, positions 28397 to 28418, positions 28509 to 28538, positions 28744 to 28784, positions 28799 to 28820, positions 28946 to 28972, positions 29010 to 29031, positions 29102 to 29130, and positions 29174 to 29196 of SEQ ID NO: 1.
(5-3) The nucleic acid or the pharmaceutically acceptable salt or hydrate thereof according to (5-1), wherein the nucleic acid or the pharmaceutically acceptable salt or hydrate thereof targets a sequence in at least one target region selected from the group consisting of positions 43 to 63, positions 48 to 68, positions 16430 to 16450, positions 16825 to 16845, positions 17022 to 17042, positions 17025 to 17045, positions 17026 to 17046, positions 17081 to 17101, positions 17255 to 17275, positions 17257 to 17277, positions 17565 to 17585, positions 17567 to 17587, positions 28746 to 28766, positions 28749 to 28769, positions 28752 to 28772, and positions 28952 to 28972 of SEQ ID NO: 1.
(6) The nucleic acid or the pharmaceutically acceptable salt or hydrate thereof according to any of (1) to (5-3), wherein the nucleic acid is siRNA or shRNA.
(7-1) The nucleic acid or the pharmaceutically acceptable salt or hydrate thereof according to (6), comprising
   a sense strand comprising
      (a) a base sequence selected from the group consisting of SEQ ID NOs: 2 to 60,
      (b) a base sequence having addition, deletion, or substitution of one or several base(s) in the base sequence selected from the group consisting of SEQ ID NOs: 2 to 60, or
      (c) a base sequence having at least 80% sequence identity to the base sequence selected from the group consisting of SEQ ID NOs: 2 to 60, and
   an antisense strand comprising a base sequence complementary to any of the sequences (a) to (c), wherein the nucleic acid or the pharmaceutically acceptable salt or hydrate thereof inhibits a function of the target region.
(7-2) The nucleic acid or the pharmaceutically acceptable salt or hydrate thereof according to (7-1), comprising
   a sense strand comprising a base sequence selected from the group consisting of SEQ ID NOs: 2 to 60, and
   an antisense strand comprising a base sequence complementary to the base sequence selected from the group consisting of SEQ ID NOs: 2 to 60.
(8-1) The nucleic acid or the pharmaceutically acceptable salt or hydrate thereof according to (7-1), comprising
   a sense strand comprising
      (a) a base sequence selected from the group consisting of SEQ ID NOs: 2, 3, 4, 5, 8, 9, 11 to 19, 28, 29, 34 to 37, 39, 40, 42, 49, 55, and 58 to 60,
      (b) a base sequence having addition, deletion, or substitution of one or several base(s) in the base sequence selected from the group consisting of SEQ ID NOs: 2, 3, 4, 5, 8, 9, 11 to 19, 28, 29, 34 to 37, 39, 40, 42, 49, 55, and 58 to 60, or
      (c) a base sequence having at least 80% sequence identity to the base sequence selected from the group consisting of SEQ ID NOs: 2, 3, 4, 5, 8, 9, 11 to 19, 28, 29, 34 to 37, 39, 40, 42, 49, 55, and 58 to 60, and
   an antisense strand comprising a base sequence complementary to any of the sequences (a) to (c),
   wherein the nucleic acid or the pharmaceutically acceptable salt or hydrate thereof inhibits a function of the target region.
(8-2) The nucleic acid or the pharmaceutically acceptable salt or hydrate thereof according to (8-1), wherein the nucleic acid comprises a sense strand comprising a base sequence selected from the group consisting of SEQ ID NOs: 2, 3, 4, 5, 8, 9, 11 to 19, 28, 29, 34 to 37, 39, 40, 42, 49, 55, and 58 to 60, and an antisense strand comprising a base sequence complementary to the base sequence selected from the group consisting of SEQ ID NOs: 2, 3, 4, 5, 8, 9, 11 to 19, 28, 29, 34 to 37, 39, 40, 42, 49, 55, and 58 to 60.
(9-1) The nucleic acid or the pharmaceutically acceptable salt or hydrate thereof according to (8-1), comprising
   a sense strand comprising
      (a) a base sequence selected from the group consisting of SEQ ID NOs: 3, 35, 36, 58, and 59,
      (b) a base sequence having addition, deletion, or substitution of one or several base(s) in the base sequence selected from the group consisting of SEQ ID NOs: 3, 35, 36, 58, and 59, or
      (c) a base sequence having at least 80% sequence identity to the base sequence selected from the group consisting of SEQ ID NOs: 3, 35, 36, 58, and 59, and
   an antisense strand comprising a base sequence complementary to any of the sequences (a) to (c),
   wherein the nucleic acid or the pharmaceutically acceptable salt or hydrate thereof inhibits a function of the target region.
(9-2) The nucleic acid or the pharmaceutically acceptable salt or hydrate thereof according to (9-1), comprising
   a sense strand comprising a base sequence selected from the group consisting of SEQ ID NOs: 3, 35, 36, 58, and 59, and
   an antisense strand comprising a base sequence complementary to the base sequence selected from the group consisting of 3, 35, 36, 58, and 59.
(10) The nucleic acid or the pharmaceutically acceptable salt or hydrate thereof according to any of (1) to (9-2), wherein the sugar moiety and/or the phosphate bond moiety of at least one nucleotide constituting the nucleic acid are/is modified.
(11) The nucleic acid or the pharmaceutically acceptable salt or hydrate thereof according to any of (1) to (10), wherein
   at least one nucleotide constituting the nucleic acid has a ribose, and the 2'-OH group of the ribose is replaced by F or OCH₃, and/or
   the nucleic acid is siRNA having an overhang, the sugar moiety of a nucleotide of the overhang moiety comprises a deoxyribose, and the 2'-H group of the deoxyribose is optionally replaced by F or OCH₃.
(12) A vector comprising the nucleic acid according to any of (1) to (11) or DNA encoding the nucleic acid.
(13) A pharmaceutical composition comprising the nucleic acid or the pharmaceutically acceptable salt or hydrate thereof according to any of (1) to (11) or the vector according to (12).
(14) The pharmaceutical composition according to (13) for the treatment and/or prevention of viral infection, wherein the virus is SARS-CoV-2, SARS-CoV-1, or MERS-CoV.
(15) A method for treating and/or preventing viral infection, comprising administering to a subject an effective amount of the nucleic acid or the pharmaceutically acceptable salt or hydrate thereof according to any of (1) to (11), the vector according to (12), or the pharmaceutical composition according to (13) or (14), wherein the virus is SARS-CoV-2, SARS-CoV-1, or MERS-CoV.
(16) Use of the nucleic acid or the pharmaceutically acceptable salt or hydrate thereof according to any of (1) to (11), the vector according to (12), or the pharmaceutical composition according to (13) in the manufacture of a medicament for the treatment and/or prevention of viral infection.

The present invention provides an antiviral nucleic acid against SARS-CoV-2, SARS-CoV-1, or MERS-CoV.

### Description of Embodiments

In one embodiment, the present invention relates to an antiviral nucleic acid or a pharmaceutically acceptable salt or hydrate thereof against SARS-CoV-2, SARS-CoV-1, or MERS-CoV (hereinafter, the antiviral nucleic acid or the pharmaceutically acceptable salt or hydrate thereof is also collectively referred to as the "antiviral nucleic acid, etc. according to the present invention").

The nucleic acid according to the present invention is composed of nucleotides as constituent units. The nucleotides may be any of ribonucleotides, deoxyribonucleotides and modified nucleotides.

The modified nucleotide refers to one having fully or partly modified nucleobases, sugar moieties and/or phosphate bond moieties, which constitute the ribonucleotide or deoxyribonucleotide.

As used herein, the nucleobase includes, for example, adenine, guanine, hypoxanthine, cytosine, thymine, uracil, and modified bases thereof. Examples of such modified bases include, but not limited to, pseudouracil, 3-methyluracil, dihydrouracil, 5-alkylcytosines (e.g., 5-methylcytosine), 5-alkyluracils (e.g., 5-ethyluracil), 5-halouracils (5-bromouracil), 6-azapyrimidine, 6-alkylpyrimidines (6-methyluracil), 2-thiouracil, 4-thiouracil, 4-acetylcytosine, 5-(carboxyhydroxymethyl) uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, xanthine, etc.

In one embodiment, the sugar moiety and/or the phosphate bond moiety of at least one nucleotide constituting the nucleic acid according to the present invention are/is modified.

As used herein, modification of the sugar moiety may include, for example, modifications at the 2'-position of ribose and modifications of the other positions of the sugar. The modification at the 2'-position of ribose includes a modification replacing the 2'-OH of ribose with OR, R, R'OR, SH, SR, NH₂, NHR, NR₂, N₃, CN, F, Cl, Br or I, wherein R represents an alkyl or an aryl, and R' represents an alkylene.

The modification for the other positions of the sugar includes but not limited to, for example, replacement of O at the 4' position of ribose or deoxyribose with S, bridging between 2' and 4' positions of the sugar, *e.g*., LNA (locked nucleic acid) or ENA (2'-O,4'-C-ethylene-bridged nucleic acids).

In one embodiment, at least one nucleotide constituting the nucleic acid according to the present invention has a ribose, and the 2'-OH group of the ribose is replaced by F or OCH₃. In one embodiment, the nucleic acid is siRNA having an overhang, the sugar moiety of a nucleotide of the overhang moiety comprises a deoxyribose, and the 2'-H group of the deoxyribose is optionally replaced by F or OCH₃.

A modification of the phosphate bond moiety includes, for example, a modification of replacing phosphodiester bond with phosphorothioate bond, phosphorodithioate bond, alkyl phosphonate bond, phosphoramidate bond or boranophosphate bond (Enya et al: Bioorganic & Medicinal Chemistry, 2008, 18, 9154-9160) (cf., *e.g.*, Japan Domestic Re-Publications of PCT Publication Nos. 2006/129594 and 2006/038608).

As used herein, the alkyl includes preferably a straight or branched alkyl having 1 to 6 carbon atoms. Specific examples include methyl, ethyl, *n*-propyl, isopropyl, *n*-butyl, isobutyl, *sec*-butyl, *tert*-butyl, *n-*pentyl, isopentyl, neopentyl, *tert*-pentyl, *n*-hexyl, and isohexyl. The alkyl may optionally be substituted. Examples of such substituents are a halogen, an alkoxy, cyano, and nitro. The alkyl may be substituted with 1 to 3 substituents.

As used herein, the cycloalkyl includes preferably a cycloalkyl having 5 to 12 carbon atoms. Specific examples include cyclopentyl, cyclohexyl, cycloheptyl, cyclooctyl, cyclodecyl, and cyclododecyl.

As used herein, the halogen includes fluorine, chlorine, bromine, and iodine.

The alkoxy includes a straight or branched alkoxy having 1 to 6 carbon atoms such as methoxy, ethoxy, *n-*propoxy, isopropoxy, *n*-butoxy, isobutoxy, *sec*-butoxy, *tert*-butoxy, *n*-pentyloxy, isopentyloxy, *n*-hexyloxy, isohexyloxy, etc. Among others, an alkoxy having 1 to 3 carbon atoms is preferred.

As used herein, the aryl includes preferably an aryl having 6 to 10 carbon atoms. Specific examples include phenyl, α-naphthyl, and β-naphthyl. Among others, phenyl is preferred. The aryl may optionally be substituted. Examples of such substituents are an alkyl, a halogen, an alkoxy, cyano, and nitro. The aryl may be substituted with one to three of such substituents.

As used herein, the alkylene includes preferably a straight or branched alkylene having 1 to 6 carbon atoms. Specific examples include methylene, ethylene, trimethylene, tetramethylene, pentamethylene, hexamethylene, 2-(ethyl) trimethylene, and 1-(methyl) tetramethylene.

As used herein, the acyl includes a straight or branched alkanoyl or aroyl. Examples of the alkanoyl include formyl, acetyl, 2-methylacetyl, 2,2-dimethylacetyl, propionyl, butyryl, isobutyryl, pentanoyl, 2,2-dimethylpropionyl, hexanoyl, etc. Examples of the aroyl include benzoyl, toluoyl, and naphthoyl. The aroyl may optionally be substituted at substitutable positions and may be substituted with an alkyl(s) .

The nucleic acid according to the present invention can be synthesized by a known approach (cf., *e.g*., Nucleic Acids Research, 12: 4539-4557, 1984). Also, the nucleic acid according to the present invention may be easily synthesized using various automated synthesizers (*e.g*., AKTA oligopilot plus 10/100 (GE Healthcare)). Alternatively, the synthesis may also be entrusted to a third-party organization (*e.g*., Promega Inc., Takara Co., or Japan Bio Service Co.), etc.

Examples of the pharmaceutically acceptable salt of the antiviral nucleic acid according to the present invention include alkali metal salts such as sodium salt, potassium salt, and lithium salt; alkaline earth metal salts such as calcium salt and magnesium salt; metal salts such as aluminum salt, iron salt, zinc salt, copper salt, nickel salt, cobalt salt, etc.; ammonium salts; organic amine salts such as t-octylamine salt, dibenzylamine salt, morpholine salt, glucosamine salt, phenylglycine alkyl ester salt, ethylenediamine salt, *N-*methylglucamine salt, guanidine salt, diethylamine salt, triethylamine salt, dicyclohexylamine salt, *N,N' -* dibenzylethylenediamine salt, chloroprocaine salt, procaine salt, diethanolamine salt, *N-*benzylphenethylamine salt, piperazine salt, tetramethylammonium salt, tris(hydroxymethyl)aminomethane salt; hydrohalide salts such as hydrofluoric acid salt, hydrochloride salt, hydrobromide salt, and hydroiodide salt; inorganic acid salts such as nitrate salt, perchlorate salt, sulfate salt, phosphate salt, etc.; lower alkane sulfonates such as methanesulfonate salt, trifluoromethanesulfonate salt, and ethanesulfonate salt; arylsulfonate salt such as benzenesulfonate salt and p-toluenesulfonate salt; organic acid salts such as acetate salt, malate salt, fumarate salt, succinate salt, citrate salt, tartrate salt, oxalate salt, maleate salt, etc.; and, amino acid salts such as glycine salt, lysine salt, arginine salt, ornithine salt, glutamic acid salt, and aspartic acid salt. These salts may be produced by known methods. Alternatively, the antiviral nucleic acid according to the present invention or the salt thereof may be in the form of a hydrate thereof.

In one embodiment, the antiviral nucleic acid, etc. according to the present invention has an antiviral effect on SARS-CoV-2, SARS-CoV-1 or MERS-CoV, for example, SARS-CoV-2 or SARS-CoV-1, for example, SARS-CoV-2. As used herein, the SARS-CoV-2 encompasses a virus having a genomic RNA sequence consisting of the base sequence (SEQ ID NO: 1) of NC_045512.2, or a mutant thereof. The SARS-CoV-1 encompasses a virus having a genomic RNA sequence consisting of the base sequence of NC_004718.3, or a mutant thereof. The MERS-CoV encompasses a virus having a genomic RNA sequence consisting of the base sequence of NC_019843.3, or a mutant thereof.

Herein, the mutant refers to a progeny having a new property formed by a mutation in which genetic information is partially changed due to partial misreading or recombination in the course of replication of a viral gene. Although the property of the mutant is partially changed under the influence of the changed genetic information, the original species of the virus is not changed. As for the family *Coronaviridae,* it is reported that viruses that share 90% or more of the amino acid sequence of conserved replicase region belong to the same viral species (cf., the section Coronaviridae of Nidovirales of ICTV 9th report (2011) (https://talk.ictvonline.org/ictv-reports/ictv_9th_report/positive-sense-rna-viruses-2011/w/posrna_viruses/222/coronaviridae)).

In one embodiment, the antiviral effect means an effect of suppressing the growth of a virus and/or reducing the infectivity of the virus. Whether or not the antiviral nucleic acid, etc. according to the present invention has the antiviral effect can be tested as described in Examples herein. For example, the presence or absence of the antiviral effect can be measured by preparing a plasmid for expression of a nucleic acid comprising a target sequence, introducing the plasmid and the antiviral nucleic acid, etc. according to the present invention into a cell, and examining whether or not the amount of the nucleic acid comprising the target sequence expressed from the cell is decreased (*e.g*., by 5% or more, 10% or more, or 20% or more) as compared with the case of introducing a negative control nucleic acid. Alternatively, the presence or absence of the antiviral effect can be measured by introducing a virus and the antiviral nucleic acid, etc. according to the present invention into a cell, culturing the cell, and then examining whether or not the amount of the virus or a nucleic acid derived therefrom in the cell or in a cell culture supernatant is decreased (*e.g*., by 5% or more, 10% or more, or 20% or more) as compared with the case of introducing a negative control nucleic acid.

The antiviral nucleic acid, etc. according to the present invention targets a sequence in at least one target region selected from the group consisting of 5' UTR region, nsp3 region, 3C-like proteinase region, nsp9 region, RNA-dependent RNA polymerase region, helicase region, 3'-to-5' exonuclease region, 2'-O-ribose methyltransferase region, S region including S1 region and S2 region, E region, M region, and N region in genomic RNA of SARS-CoV-2.

The sequence of the genomic RNA of SARS-CoV-2 and the sequence of each region in the genomic RNA can be easily determined with reference to a database (*e.g*., NCBI). The sequence of the genomic RNA of SARS-CoV-2 can be, for example, the base sequence (SEQ ID NO: 1) of NC_045512.2. In the base sequence of SEQ ID NO: 1, the 5' UTR region can be a base sequence from positions 1 to 265 of SEQ ID NO: 1; the nsp3 region can be a base sequence from positions 2720 to 8554 of SEQ ID NO: 1; the 3C-like proteinase region can be a base sequence from positions 10055 to 10972 of SEQ ID NO: 1; the nsp9 region can be a base sequence from positions 12686 to 13024 of SEQ ID NO: 1; the RNA-dependent RNA polymerase region can be a base sequence from positions 13442 to 16236 of SEQ ID NO: 1; the helicase region can be a base sequence from positions 16237 to 18039 of SEQ ID NO: 1; the 3'-to-5' exonuclease region can be a base sequence from positions 18040 to 19620 of SEQ ID NO: 1; the 2'-O-ribose methyltransferase region can be a base sequence from positions 20659 to 21552 of SEQ ID NO: 1; the S region can be a base sequence from positions 21563 to 25384 of SEQ ID NO: 1 (the S1 region can be a base sequence from positions 21599 to 23617 of SEQ ID NO: 1, and the S2 region can be a base sequence from positions 23618 to 25381 of SEQ ID NO: 1); the E region can be a base sequence from positions 26245 to 26472 of SEQ ID NO: 1; the M region can be a base sequence from positions 26523 to 27191 of SEQ ID NO: 1; and the N region can be a base sequence from positions 28274 to 29533 of SEQ ID NO: 1.

The length of the target sequence is not limited and can be, for example, a sequence of 16 or more consecutive bases, 17 or more consecutive bases, 18 or more consecutive bases, 19 or more consecutive bases, or 20 or more consecutive bases in the target region and can be a sequence of 30 or less bases, 28 or less bases, 26 or less bases, 24 or less bases, or 22 or less bases. In one embodiment, the target sequence is a sequence of 16 to 30 consecutive bases, 18 to 26 consecutive bases, 20 to 22 consecutive bases, or 21 consecutive bases in the target region.

In one embodiment, the antiviral nucleic acid, etc. according to the present invention targets a conserved region in the sequences of genomic RNA of SARS-CoV-2 and genomic RNA of SARS-CoV-1 and therefore may exert an antiviral effect on the genus *Betacoronavirus* including SARS-CoV-2, SARS-CoV-1 and MERS-CoV. The antiviral nucleic acid, etc. according to the present invention targets, for example, a sequence in at least one target region selected from the group consisting of positions 43 to 69, positions 242 to 279, positions 3352 to 3372, positions 6406 to 6427, positions 10406 to 10431, positions 10484 to 10506, positions 12839 to 12867, positions 12898 to 12922, positions 12965 to 12990, positions 13481 to 13502, positions 13762 to 13790, positions 13894 to 13916, positions 14290 to 14312, positions 14654 to 14687, positions 14750 to 14777, positions 14824 to 14846, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15622 to 15644, positions 15829 to 15859, positions 15929 to 15950, positions 15985 to 16011, positions 16051 to 16085, positions 16189 to 16220, positions 16430 to 16451, positions 16822 to 16845, positions 17015 to 17051, positions 17080 to 17103, positions 17254 to 17277, positions 17564 to 17600, positions 18196 to 18218, positions 18253 to 18278, positions 19568 to 19595, positions 20888 to 20909, positions 21502 to 21524, positions 22814 to 22836, positions 23093 to 23113, positions 23956 to 23976, positions 24302 to 24324, positions 24467 to 24489, positions 24620 to 24651, positions 24662 to 24684, positions 24962 to 24982, positions 25104 to 25128, positions 25364 to 25384, positions 26287 to 26325, positions 26574 to 26604, positions 28274 to 28294, positions 28397 to 28418, positions 28509 to 28538, positions 28744 to 28784, positions 28799 to 28820, positions 28946 to 28972, positions 29010 to 29031, positions 29102 to 29130, and positions 29174 to 29196 of SEQ ID NO: 1.

In one embodiment, the antiviral nucleic acid, etc. according to the present invention targets a sequence consisting of 21 consecutive bases in the target region, for example, at least one target region selected from the group consisting of positions 43 to 63, positions 44 to 64, positions 45 to 65, positions 46 to 66, positions 47 to 67, positions 48 to 68, positions 49 to 69, positions 242 to 262, positions 243 to 263, positions 244 to 264, positions 245 to 265, positions 246 to 266, positions 247 to 267, positions 248 to 268, positions 249 to 269, positions 250 to 270, positions 251 to 271, positions 252 to 272, positions 253 to 273, positions 254 to 274, positions 255 to 275, positions 256 to 276, positions 257 to 277, positions 258 to 278, positions 259 to 279, positions 3352 to 3372, positions 6406 to 6426, positions 6407 to 6427, positions 10406 to 10426, positions 10407 to 10427, positions 10408 to 10428, positions 10409 to 10429, positions 10410 to 10430, positions 10411 to 10431, positions 10484 to 10504, positions 10485 to 10505, positions 10486 to 10506, positions 12839 to 12859, positions 12840 to 12860, positions 12841 to 12861, positions 12842 to 12862, positions 12843 to 12863, positions 12844 to 12864, positions 12845 to 12865, positions 12846 to 12866, positions 12847 to 12867, positions 12898 to 12918, positions 12899 to 12919, positions 12900 to 12920, positions 12901 to 12921, positions 12902 to 12922, positions 12965 to 12985, positions 12966 to 12986, positions 12967 to 12987, positions 12968 to 12988, positions 12969 to 12989, positions 12970 to 12990, positions 13481 to 13501, positions 13482 to 13502, positions 13762 to 13782, positions 13763 to 13783, positions 13764 to 13784, positions 13765 to 13785, positions 13766 to 13786, positions 13767 to 13787, positions 13768 to 13788, positions 13769 to 13789, positions 13770 to 13790, positions 13894 to 13914, positions 13895 to 13915, positions 13896 to 13916, positions 14290 to 14310, positions 14291 to 14311, positions 14292 to 14312, positions 14654 to 14674, positions 14655 to 14675, positions 14656 to 14676, positions 14657 to 14677, positions 14658 to 14678, positions 14659 to 14679, positions 14660 to 14680, positions 14661 to 14681, positions 14662 to 14682, positions 14663 to 14683, positions 14664 to 14684, positions 14665 to 14685, positions 14666 to 14686, positions 14667 to 14687, positions 14750 to 14770, positions 14751 to 14771, positions 14752 to 14772, positions 14753 to 14773, positions 14754 to 14774, positions 14755 to 14775, positions 14756 to 14776, positions 14757 to 14777, positions 14824 to 14844, positions 14825 to 14845, positions 14826 to 14846, positions 14878 to 14898, positions 14879 to 14899, positions 14880 to 14900, positions 14881 to 14901, positions 14882 to 14902, positions 14883 to 14903, positions 14884 to 14904, positions 14885 to 14905, positions 14886 to 14906, positions 14887 to 14907, positions 14888 to 14908, positions 14889 to 14909, positions 14953 to 14973, positions 14954 to 14974, positions 14955 to 14975, positions 14956 to 14976, positions 14957 to 14977, positions 14958 to 14978, positions 14959 to 14979, positions 14960 to 14980, positions 14961 to 14981, positions 14962 to 14982, positions 14963 to 14983, positions 14964 to 14984, positions 14965 to 14985, positions 14966 to 14986, positions 14967 to 14987, positions 14968 to 14988, positions 14969 to 14989, positions 14970 to 14990, positions 14992 to 15012, positions 14993 to 15013, positions 14994 to 15014, positions 14995 to 15015, positions 14996 to 15016, positions 14997 to 15017, positions 14998 to 15018, positions 14999 to 15019, positions 15000 to 15020, positions 15001 to 15021, positions 15002 to 15022, positions 15003 to 15023, positions 15004 to 15024, positions 15005 to 15025, positions 15006 to 15026, positions 15037 to 15057, positions 15038 to 15058, positions 15039 to 15059, positions 15040 to 15060, positions 15041 to 15061, positions 15063 to 15083, positions 15064 to 15084, positions 15065 to 15085, positions 15066 to 15086, positions 15067 to 15087, positions 15068 to 15088, positions 15069 to 15089, positions 15070 to 15090, positions 15071 to 15091, positions 15072 to 15092, positions 15073 to 15093, positions 15074 to 15094, positions 15075 to 15095, positions 15076 to 15096, positions 15077 to 15097, positions 15078 to 15098, positions 15079 to 15099, positions 15080 to 15100, positions 15081 to 15101, positions 15082 to 15102, positions 15083 to 15103, positions 15084 to 15104, positions 15085 to 15105, positions 15086 to 15106, positions 15087 to 15107, positions 15088 to 15108, positions 15089 to 15109, positions 15090 to 15110, positions 15091 to 15111, positions 15092 to 15112, positions 15093 to 15113, positions 15094 to 15114, positions 15095 to 15115, positions 15096 to 15116, positions 15097 to 15117, positions 15098 to 15118, positions 15099 to 15119, positions 15100 to 15120, positions 15101 to 15121, positions 15102 to 15122, positions 15103 to 15123, positions 15104 to 15124, positions 15105 to 15125, positions 15106 to 15126, positions 15107 to 15127, positions 15108 to 15128, positions 15109 to 15129, positions 15110 to 15130, positions 15111 to 15131, positions 15112 to 15132, positions 15113 to 15133, positions 15114 to 15134, positions 15115 to 15135, positions 15116 to 15136, positions 15117 to 15137, positions 15118 to 15138, positions 15119 to 15139, positions 15120 to 15140, positions 15172 to 15192, positions 15173 to 15193, positions 15174 to 15194, positions 15175 to 15195, positions 15176 to 15196, positions 15177 to 15197, positions 15178 to 15198, positions 15278 to 15298, positions 15279 to 15299, positions 15454 to 15474, positions 15455 to 15475, positions 15456 to 15476, positions 15457 to 15477, positions 15458 to 15478, positions 15459 to 15479, positions.15496 to 15516, positions 15497 to 15517, positions 15498 to 15518, positions 15622 to 15642, positions 15623 to 15643, positions 15624 to 15644, positions 15829 to 15849, positions 15830 to 15850, positions 15831 to 15851, positions 15832 to 15852, positions 15833 to 15853, positions 15834 to 15854, positions 15835 to 15855, positions 15836 to 15856, positions 15837 to 15857, positions 15838 to 15858, positions 15839 to 15859, positions 15929 to 15949, positions 15930 to 15950, positions 15985 to 16005, positions 15986 to 16006, positions 15987 to 16007, positions 15988 to 16008, positions 15989 to 16009, positions 15990 to 16010, positions 15991 to 16011, positions 16051 to 16071, positions 16052 to 16072, positions 16053 to 16073, positions 16054 to 16074, positions 16055 to 16075, positions 16056 to 16076, positions 16057 to 16077, positions 16058 to 16078, positions 16059 to 16079, positions 16060 to 16080, positions 16061 to 16081, positions 16062 to 16082, positions 16063 to 16083, positions 16064 to 16084, positions 16065 to 16085, positions 16189 to 16209, positions 16190 to 16210, positions 16191 to 16211, positions 16192 to 16212, positions 16193 to 16213, positions 16194 to 16214, positions 16195 to 16215, positions 16196 to 16216, positions 16197 to 16217, positions 16198 to 16218, positions 16199 to 16219, positions 16200 to 16220, positions 16430 to 16450, positions 16431 to 16451, positions 16822 to 16842, positions 16823 to 16842, positions 16823 to 16843, positions 16824 to 16844, positions 16825 to 16845, positions 17015 to 17035, positions 17016 to 17036, positions 17017 to 17037, positions 17018 to 17038, positions 17019 to 17039, positions 17020 to 17040, positions 17021 to 17041, positions 17022 to 17042, positions 17023 to 17043, positions 17024 to 17044, positions 17025 to 17045, positions 17026 to 17046, positions 17027 to 17047, positions 17028 to 17048, positions 17029 to 17049, positions 17030 to 17050, positions 17031 to 17051, positions 17080 to 17100, positions 17081 to 17101, positions 17082 to 17102, positions 17083 to 17103, positions 17254 to 17274, positions 17255 to 17275, positions 17256 to 17276, positions 17257 to 17277, positions 17564 to 17584, positions 17565 to 17585, positions 17566 to 17586, positions 17567 to 17587, positions 17568 to 17588, positions 17569 to 17589, positions 17570 to 17590, positions 17571 to 17591, positions 17572 to 17592, positions 17573 to 17593, positions 17574 to 17594, positions 17575 to 17595, positions 17576 to 17596, positions 17577 to 17597, positions 17578 to 17598, positions 17579 to 17599, positions 17580 to 17600, positions 18196 to 18216, positions 18197 to 18217, positions 18198 to 18218, positions 18253 to 18273, positions 18254 to 18274, positions 18255 to 18275, positions 18256 to 18276, positions 18257 to 18277, positions 18258 to 18278, positions 19568 to 19588, positions 19569 to 19589, positions 19570 to 19590, positions 19571 to 19591, positions 19572 to 19592, positions 19573 to 19593, positions 19574 to 19594, positions 19575 to 19595, positions 20888 to 20908, positions 20889 to 20909, positions 21502 to 21522, positions 21503 to 21523, positions 21504 to 21524, positions 22814 to 22834, positions 22815 to 22835, positions 22816 to 22836, positions 23093 to 23113, positions 23956 to 23976, positions 24302 to 24322, positions 24303 to 24333, positions 24304 to 24324, positions 24467 to 24487, positions 24468 to 24488, positions 24469 to 24489, positions 24620 to 24640, positions 24621 to 24641, positions 24622 to 24642, positions 24623 to 24643, positions 24624 to 24644, positions 24625 to 24645, positions 24626 to 24646, positions 24627 to 24647, positions 24628 to 24648, positions 24629 to 24649, positions 24630 to 24650, positions 24631 to 24651, positions 24662 to 24682, positions 24663 to 24683, positions 24664 to 24684, positions 24962 to 24982, positions 25104 to 25124, positions 25105 to 25125, positions 25106 to 25126, positions 25107 to 25127, positions 25108 to 25128, positions 25364 to 25384, positions 26287 to 26307, positions 26288 to 26308, positions 26289 to 26309, positions 26290 to 26310, positions 26291 to 26311, positions 26292 to 26312, positions 26293 to 26313, positions 26294 to 26314, positions 26295 to 26315, positions 26296 to 26316, positions 26297 to 26317, positions 26298 to 26318, positions 26299 to 26319, positions 26300 to 26320, positions 26301 to 26321, positions 26302 to 26322, positions 26303 to 26323, positions 26304 to 26324, positions 26305 to 26325, positions 26574 to 26594, positions 26575 to 26595, positions 29576 to 29596, positions 26577 to 26597, positions 26578 to 26598, positions 26579 to 26599, positions 26580 to 26600, positions 26581 to 26601, positions 26582 to 26602, positions 26583 to 26603, positions 26584 to 26604, positions 28273 to 28293, positions 28274 to 28294, positions 28397 to 28417, positions 28398 to 28418, positions 28509 to 28529, positions 28510 to 28530, positions 28511 to 28531, positions 28512 to 28532, positions 28513 to 28533, positions 28514 to 28534, positions 28515 to 28535, positions 28516 to 28536, positions 28517 to 28537, positions 28518 to 28538, positions 28744 to 288764, positions 28745 to 28765, positions 28746 to 28766, positions 28747 to 28767, positions 28748 to 28768, positions 28749 to 28769, positions 28750 to 28770, positions 28751 to 28771, positions 28752 to 28772, positions 28753 to 28773, positions 28754 to 28774, positions 28755 to 28775, positions 28756 to 28776, positions 28757 to 28777, positions 28758 to 28778, positions 28759 to 28779, positions 28760 to 28780, positions 28761 to 28781, positions 28762 to 28782, positions 28763 to 28783, positions 28764 to 28784, positions 28799 to 28819, positions 28800 to 28820, positions 28946 to 28966, positions 28947 to 28967, positions 28948 to 28968, positions 28949 to 28969, positions 28950 to 28970, positions 28951 to 28971, positions 28952 to 28972, positions 29010 to 29030, positions 29011 to 29031, positions 29102 to 29122, positions 29103 to 29123, positions 29104 to 29124, positions 29105 to 29125, positions 29106 to 29126, positions 29107 to 29127, positions 29108 to 29128, positions 29109 to 29129, positions 29110 to 29130, positions 29174 to 29194, position 29175, and positions 29176 to 29196 of region SEQ ID NO: 1, or a sequence in this target region.

In one embodiment, the antiviral nucleic acid, etc. according to the present invention targets at least one target region selected from the group consisting of positions 43 to 63, positions 48 to 68, positions 3352 to 3372, positions 6406 to 6426, positions 10410 to 10430, positions 10411 to 10431, positions 10486 to 10506, positions 12898 to 12918, positions 13763 to 13783, positions 13765 to 13785, positions 13895 to 13915, positions 14883 to 14903, positions 14887 to 14907, positions 14955 to 15015, positions 15002 to 15022, positions 15077 to 15097, positions 15079 to 15099, positions 15105 to 15125, positions 15107 to 15127, positions 15110 to 15130, positions 15113 to 15133, positions 15116 to 15136, positions 15498 to 15518, positions 15623 to 15643, positions 15838 to 15858, positions 15987 to 16007, positions 16061 to 16081, positions 16063 to 16083, positions 16193 to 16213, positions 16430 to 16450, positions 16825 to 16845, positions 17022 to 17042, positions 17025 to 17045, positions 17026 to 17046, positions 17081 to 17101, positions 17255 to 17275, positions 17257 to 17277, positions 17565 to 17585, positions 17567 to 17587, positions 19570 to 19590, positions 21504 to 21524, positions 22815 to 22835, positions 22816 to 22836, positions 23956 to 23976, positions 24303 to 24323, positions 24624 to 24644, positions 24625 to 24645, positions 24630 to 24650, positions 24664 to 24684, positions 25364 to 25384, positions 26291 to 26311, positions 26303 to 26323, positions 26579 to 26599, positions 26583 to 26603, positions 26584 to 26604, positions 28746 to 28766, positions 28749 to 28769, positions 28752 to 28772, and positions 28952 to 28972 of SEQ ID NO: 1, or a sequence in this target region.

In one embodiment, the antiviral nucleic acid, etc. according to the present invention targets at least one target region selected from the group consisting of positions 43 to 63, positions 48 to 68, positions 3352 to 3372, positions 6406 to 6426, positions 10486 to 10506, positions 12898 to 12918, positions 13765 to 13785, positions 13895 to 13915, positions 14883 to 14903, positions 14887 to 14907, positions 14955 to 15015, positions 15002 to 15022, positions 15077 to 15097, positions 15079 to 15099, positions 15105 to 15125, positions 16061 to 16081, positions 16063 to 16083, positions 17025 to 17045, positions 17026 to 17046, positions 17081 to 17101, positions 17255 to 17275, positions 17565 to 17585, positions 17567 to 17587, positions 21504 to 21524, positions 24630 to 24650, positions 26583 to 26603, positions 28749 to 28769, positions 28752 to 28772, and positions 28952 to 28972 of SEQ ID NO: 1, or a sequence in this target region.

In one embodiment, the antiviral nucleic acid, etc. according to the present invention targets a sequence in at least one target region selected from the group consisting of 5' UTR region, helicase region, and N region in genomic RNA of SARS-CoV-2.

In one embodiment, the antiviral nucleic acid, etc. according to the present invention targets a conserved region in the sequences of genomic RNA of SARS-CoV-2 and genomic RNA of SARS-CoV-1, for example, a sequence in at least one target region selected from the group consisting of positions 43 to 69, positions 16430 to 16451, positions 16822 to 16845, positions 17015 to 17051, positions 17080 to 17103, positions 17254 to 17277, positions 17564 to 17600, positions 28274 to 28294, positions 28397 to 28418, positions 28509 to 28538, positions 28744 to 28784, positions 28799 to 28820, positions 28946 to 28972, positions 29010 to 29031, positions 29102 to 29130, and positions 29174 to 29196 of SEQ ID NO: 1.

In one embodiment, the antiviral nucleic acid, etc. according to the present invention targets a sequence consisting of 21 consecutive bases in the target region, for example, at least one target region selected from the group consisting of positions 43 to 63, positions 48 to 68, positions 16430 to 16450, positions 16825 to 16845, positions 17022 to 17042, positions 17025 to 17045, positions 17026 to 17046, positions 17081 to 17101, positions 17255 to 17275, positions 17257 to 17277, positions 17565 to 17585, positions 17567 to 17587, positions 28746 to 28766, positions 28749 to 28769, positions 28752 to 28772, and positions 28952 to 28972 of region SEQ ID NO: 1, or a sequence in this target region.

In one embodiment, the nucleic acid according to the present invention is siRNA or shRNA, or DNA encoding it. The siRNA refers to double-stranded RNA that comprises a sense strand and an antisense strand and can induce RNA interference in a sequence-specific manner. As used herein, the RNA interference refers to a phenomenon in which a nucleic acid having a target sequence is degraded in a sequence-specific manner by double-stranded RNA. The shRNA is hairpin RNA comprising a sense strand, an antisense strand, and a single-stranded loop sequence (hairpin sequence) linking the sense strand and the antisense strand through a covalent bond. The shRNA, when introduced into a cell, is processed by dicer to form siRNA. Examples of the DNA encoding the siRNA include tandem DNA comprising two promoters (*e.g*., U6 promoters), a base sequence encoding a sense strand, and a base sequence encoding an antisense.

As used herein, the term "sense strand" refers to a nucleotide strand partially or completely complementary to at least a portion of a corresponding antisense strand. The sense strand can comprise a nucleic acid sequence having homology to a target sequence. As used herein, the term "antisense strand" refers to a nucleotide strand partially or completely complementary to at least a portion of a target nucleic acid sequence.

The 3' end of each of sense RNA and antisense RNA may have an overhang of 2 to 5 nucleotides, for example, 2 nucleotides. It is pointed out that the overhang may interact with RISC. The overhang contributes to improvement in stability against degradation by nuclease and however, does not influence specificity for a target sequence. Therefore, any sequence (*e.g*., a poly T sequence) can be used.

The length of the nucleic acid according to the present invention is not limited. For example, siRNA can have a sequence of 16 bp or longer, 17 bp or longer, 18 bp or longer, 19 bp or longer, or 20 bp or longer and can have a sequence of 30 bp or shorter, 28 bp or shorter, 26 bp or shorter, 24 bp or shorter, or 22 bp or shorter. In one embodiment, the nucleic acid has a sequence of 16 to 30 consecutive bp, 18 bp to 26 consecutive bp, 20 to 22 consecutive bp, or 21 consecutive bp in the target region. In the case of shRNA, the base length of the nucleic acid can be a length of the double-stranded sequence plus a stem-loop (*e.g*., of 2 to 15 bases) sequence and can be, for example, 34 to 75 bases long.

The siRNA or the shRNA can be designed on the basis of the target sequence. For example, when the target sequence is a sequence consisting of 21 consecutive bases in the target region, a nucleic acid comprising a sequence consisting of 5'-terminal 19 bases of the sequence, and an overhang can be used as a sense strand. A sequence complementary to the sequence consisting of 21 consecutive bases in the target region can be used as an antisense strand (provided that when a 3'-terminal side (*e.g*., 3'-terminal 2 bases) is an overhang, the overhang can have any sequence).

In one embodiment, the siRNA or the shRNA according to the present invention comprises a sense strand comprising (a) a base sequence selected from the group consisting of SEQ ID NOs: 2 to 60, (b) a base sequence having addition, deletion, or substitution of one or several base(s) in the base sequence selected from the group consisting of SEQ ID NOs: 2 to 60, or (c) a base sequence having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the base sequence selected from the group consisting of SEQ ID NOs: 2 to 60, and an antisense strand comprising a base sequence complementary to any of the sequences (a) to (c) in the sense strand.

As used herein, the term "several" for the base sequence having addition, deletion, or substitution of one or several base(s) means 2, 3, 4, 5, 6, 7, 8, 9, or 10.

As used herein, the term "identity" between base sequences means the ratio of matched bases when two base sequences are aligned. The sequence identity can be determined using FASTA (Science 227 (4693): 1435-1441, (1985)) or algorithm BLAST (Basic Local Alignment Search Tool) by Karlin and Altschul (Proc. Natl. Acad. Sci. USA 872264-2268, 1990; and Proc Natl Acad Sci USA 90: 5873, 1993). Programs called blastn, blastx, tblastn and tblastx based on the BLAST algorithm have been developed (Altschul SF, et al: J. Mol. Biol. 215: 403, 1990). When a base sequence is analyzed using blastn, the parameters are, for example, score = 100 and wordlength = 12. When BLAST and Gapped BLAST programs are used, the default parameters for each program are employed.

In one embodiment, the siRNA or the shRNA according to the present invention comprises a sense strand comprising (a) a base sequence selected from the group consisting of SEQ ID NOs: 2, 3, 4, 5, 8, 9, 11 to 19, 28, 29, 34 to 37, 39, 40, 42, 49, 55, and 58 to 60, (b) a base sequence having addition, deletion, or substitution of one or several base(s) in the base sequence selected from the group consisting of SEQ ID NOs: 2, 3, 4, 5, 8, 9, 11 to 19, 28, 29, 34 to 37, 39, 40, 42, 49, 55, and 58 to 60, or (c) a base sequence having at least 880%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the base sequence selected from the group consisting of SEQ ID NOs: 2, 3, 4, 5, 8, 9, 11 to 19, 28, 29, 34 to 37, 39, 40, 42, 49, 55, and 58 to 60, and an antisense strand comprising a base sequence complementary to any of the sequences (a) to (c) in the sense strand.

In one embodiment, the siRNA or the shRNA according to the present invention comprises a sense strand comprising (a) a base sequence selected from the group consisting of SEQ ID NOs: 3, 35, 36, 58, and 59, (b) a base sequence having addition, deletion, or substitution of one or several base(s) in the base sequence selected from the group consisting of SEQ ID NOs: 3, 35, 36, 58, and 59, or (c) a base sequence having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the base sequence selected from the group consisting of SEQ ID NOs: 3, 35, 36, 58, and 59, and an antisense strand comprising a base sequence complementary to any of the sequences (a) to (c) in the sense strand.

In one embodiment, the sense strand in the siRNA or the shRNA according to the present invention comprises any of the sequences (a) to (c) and an overhang, or consists of them. In one embodiment, the antisense strand in the siRNA or the shRNA according to the present invention comprises a base sequence complementary to any of the sequences (a) to (c) in the sense strand and an overhang, or consists of them.

In one embodiment, the siRNA or the shRNA according to the present invention inhibits a function of the target region. As used herein, the phrase "inhibit a function of the target region" includes one or more of cleavage of genomic RNA and/or subgenomic RNA comprising the target region through binding to the target region, inhibition of the replication of genomic RNA and/or subgenomic RNA comprising the target region, inhibition of the transcription of genomic RNA and/or subgenomic RNA comprising the target region, and inhibition of the translation of the target region when the target region is translated. As used herein, the term "subgenomic RNA" means RNA that is shorter than genomic RNA, is synthesized by RNA-dependent RNA polymerase using as a template a portion of (-)-strand RNA synthesized by RNA-dependent RNA polymerase with (+)-strand genomic RNA as a template, and works as mRNA for viral protein synthesis (translation).

The siRNA or the shRNA according to the present invention may comprise a plurality of sense strand sequences and antisense strand sequences that target different regions. For example, the sense strand in the siRNA according to the present invention may comprise two or more of the sequences (a) to (c), and the antisense strand in the siRNA according to the present invention may comprise two or more base sequences complementary to the sequences (a) to (c) in the sense strand.

In one embodiment, the present invention relates to a vector comprising the nucleic acid according to the present invention or DNA encoding the nucleic acid. The vector can be prepared in a conventional manner. For example, the vector comprising tandem DNA encoding the siRNA according to the present invention can be prepared by amplifying a nucleic acid comprising a sense strand sequence and an antisense sequence by PCT using primers, cleaving the amplified fragment with restriction enzymes, and then inserting the resultant to downstream of a promoter (e.g., U6 promoter) of a vector. The vector comprising DNA encoding the shRNA according to the present invention can be prepared by synthesizing and then annealing a nucleic acid comprising a sense strand sequence, a loop sequence, and an antisense strand sequence, and inserting the resultant to downstream of a promoter (e.g., U6 promoter) of a vector. Examples of the vector include plasmids, virus vectors (*e.g*., adeno-associated virus, retrovirus, and lentivirus), and artificial chromosomes, etc.

In one embodiment, the present invention relates to a pharmaceutical composition comprising the nucleic acid, etc. or the vector according to the present invention. The pharmaceutical composition according to the present invention may comprise a plurality of nucleic acids, etc. or vectors according to the present invention.

The pharmaceutical composition according to the present invention may comprise a pharmaceutically acceptable carrier (excipient, bulking agent, binder, lubricant, etc.) and/or a known additive (buffer, isotonizing agent, chelating agent, colorant, preservative, fragrance, flavor, sweetener, etc.) in addition to the nucleic acid, etc. or the vector according to the present invention.

The pharmaceutical composition according to the present invention may comprise the nucleic acid, etc. or the vector according to the present invention in the form of a liposome with a lipid such as a cationic lipid or a neutral lipid, or the nucleic acid, etc. or the vector according to the present invention linked to a lipid such as a cationic lipid or a neutral lipid through a covalent bond.

Administration route for the composition according to the present invention is not particularly limited so long as it is pharmaceutically acceptable route for administration, and can be chosen depending upon method of treatment. Examples thereof include intratracheal administration, intravenous administration, intraarterial administration, intramuscular administration, subcutaneous administration, oral administration, tissue administration, transdermal administration, etc. Also, dosage forms which are available for the composition of the present invention are not particularly limited, and include, for example, inhalations, various injections, oral agents, drips, ointments, lotions, etc.

The administration route for the pharmaceutical composition according to the present invention is preferably intratracheal administration, and the dosage forms which are available for the composition of the present invention are preferably inhalations, specifically, for example, liquid inhalations (e.g., administered using a nebulizer), powder inhalations (e.g., administered using DPI (dry powder inhaler)), or aerosols, and preferably liquid inhalations.

A dose of the composition according to the present invention to be administered may be adjusted by taking the following factors into account: the type of the comprised according to the present invention; the dosage form of the composition; patients' conditions including age, body weight, etc.; administration route; and the characteristics and symptoms of the disease. A daily dose calculated as the amount of the nucleic acid according to the present invention can be, for example, in a range of 0.1 mg to 10 g/human, for example, in a range of 1 mg to 1 g/human, or, for example, in a range of 10 mg to 100 mg/human. The number of times of administration and a frequency of administration are not limited, and administration can be performed, for example, once to two or three times a day at 1- to 2- or 3-day intervals. The administration may be performed only once or may be performed a total of twice including another dose after several days of the initial dose.

In one embodiment, the present invention relates to a method for treating and/or preventing viral infection, comprising administering to a subject an effective amount of the nucleic acid or the pharmaceutically acceptable salt or hydrate thereof, the vector, or the pharmaceutical composition according to the present invention, wherein the virus is SARS-CoV-2, SARS-CoV-1, or MERS-CoV. The virus is, for example, SARS-CoV-2 or SARS-CoV-1, and preferably SARS-CoV-2.

The subject to which the nucleic acid, etc., the vector, or or the pharmaceutical composition according to the present invention is administered includes, for example, mammals, for example, primates such as humans, laboratory animals such as rats, mice, and Norway rats, and livestock animals such as pigs, bovines, horses, and sheep, etc., and is preferably a human.

As used herein, the treatment of viral infection encompasses one or more of alleviation, improvement, and remission of a disease caused by the virus or symptoms thereof (e.g., one or more of dyspnea, fever, dry cough, headache, chill, and muscle pain). As used herein, the prevention of viral infection encompasses reduction of a risk resulting from a disease caused by the virus or symptoms thereof.

In one embodiment, the present invention relates to use of the nucleic acid or the pharmaceutically acceptable salt or hydrate thereof, or the vector according to the present invention in the manufacture of a medicament for the treatment and/or prevention of viral infection.

### Examples

### <Example 1: Measurement of knockdown activity of siRNA using SARS-CoV-2 expression plasmid>

The sequences of genomic RNA of SARS-CoV-2 (reference sequence; NC_045512.2) (SEQ ID NO: 1) and genomic RNA of SARS-CoV-1 (reference sequence; NC_004718.3) were compared to identify regions conserved between these two species. As regions conserved between the two species, base sequences from positions 43 to 69, positions 242 to 279, positions 3352 to 3372, positions 6406 to 6427, positions 10406 to 10431, positions 10484 to 10506, positions 12839 to 12867, positions 12898 to 12922, positions 12965 to 12990, positions 13481 to 13502, positions 13762 to 13790, positions 13894 to 13916, positions 14290 to 14312, positions 14654 to 14687, positions 14750 to 14777, positions 14824 to 14846, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15622 to 15644, positions 15829 to 15859, positions 15929 to 15950, positions 15985 to 16011, positions 16051 to 16085, positions 16189 to 16220, positions 16430 to 16451, positions 16822 to 16845, positions 17015 to 17051, positions 17080 to 17103, positions 17254 to 17277, positions 17564 to 17600, positions 18196 to 18218, positions 18253 to 18278, positions 19568 to 19595, positions 20888 to 20909, positions 21502 to 21524, positions 22814 to 22836, positions 23093 to 23113, positions 23956 to 23976, positions 24302 to 24324, positions 24467 to 24489, positions 24620 to 24651, positions 24662 to 24684, positions 24962 to 24982, positions 25104 to 25128, positions 25364 to 25384, positions 26287 to 26325, positions 26574 to 26604, positions 28274 to 28294, positions 28397 to 28418, positions 28509 to 28538, positions 28744 to 28784, positions 28799 to 28820, positions 28946 to 28972, positions 29010 to 29031, positions 29102 to 29130, and positions 29174 to 29196 of SEQ ID NO: 1 were found. Test substance siRNA Nos. 1 to 59 (comprising a sense strand consisting of base sequences represented by SEQ ID NOs: 2 to 60, respectively, and an overhang region of 2 bases, and an antisense strand consisting of base sequences represented by SEQ ID NOs: 61 to 119, respectively) were prepared, which targets sequences contained in the conserved regions. Table 1-1 below shows the target sequences and the sequences of the test substance siRNA Nos. 1 to 59, etc. The sense strand and the antisense strand each comprised an overhang region of 2 bases at the 3' end. The sequence of the overhang region of the sense strand was omitted, and the overhang region of the antisense strand was indicated in lower case. A sequence complementary to the sequence including the overhang region of the antisense strand is identical to the target sequence. In actuality, a duplex-forming moiety with the sense strand is a region important for the activity of siRNA. Therefore, the overhang region does not have to be complementary to the target sequence, and any sequence can be used.

**[Table 1-1]**

| siRNA No. | Position of target sequence in SEQ ID NO: 1 | | Function of target sequence (*1) | Sequence of sense strand | SEQ ID NO | Sequence of antisense strand | SEQ ID NO |
|---|---|---|---|---|---|---|---|
| | Start | End | | | | | |
| 1 | 43 | 63 | 5'UTR | GAUCUCUUGUAGAUCUGUU | 2 | AACAGAUCUACAAGAGAUCga | 61 |
| 2 | 43 | 68 | 5'UTR | GUUGUAGAUCUGUUCUCUA | 3 | UAGAGAACAGAUCUACAAGas | 62 |
| 3 | 3352 | 3372 | nsp3 | GUUAUUUAAAACUUACUGA | 4 | UCAGUAAGUUUUAAAUAACca | 63 |
| 4 | 6406 | 6426 | nso3 | CUGAAGAAGUAGUGGAAAA | 5 | UUUUCCACUACUUCUUCAGas | 64 |
| 5 | 10410 | 10430 | 3C-like proteinase | GGUUCACCAUCUGGUGUUU | 6 | AAACACCAGAUGGUGAACCat | 65 |
| 6 | 10411 | 10431 | 3C-like proteinase | GUUCACCAUCUGGUGUUUA | 7 | UAAACACCAGAUGGUGAACca | 66 |
| 7 | 10486 | 10506 | 3C-like proteinase | GUGGUAGUGUUGGUUUUAA | 8 | UUAAAACCAACACUAGCACat | 67 |
| 8 | 12898 | 12918 | nsp9 | CUUGUAGGUUUGUUACAGA | 9 | UCUGUAACAAACCUACAAGet | 68 |
| 9 | 13763 | 13783 | RNA-dependent RNA polymerase | GACAUGGUACCACAUAUAU | 10 | AUAUAUGUGGUACCAUGUCac | 69 |
| 10 | 13765 | 13785 | RNA-dependent RNA polymerase | CAUGGUACCACAUAUAUCA | 11 | UGAUAUAUGUGGUACCAUGtc | 70 |
| 11 | 13895 | 13915 | RNA-deoendent RNA polymerase | GAUGAUGAUUAUUUCAAUA | 12 | UAUUGAAAUAAUCAUCAUCac | 71 |
| 12 | 14883 | 14903 | RNA-dependent RNA polymerase | GUUACGAUGGUGGCUGUAU | 13 | AUACAGCCACCAUCGUAACaa | 72 |
| 13 | 14887 | 14907 | RNA-dependent RNA polymerase | CGAUGGUGGCUGUAUUAAU | 14 | AUUAAUACAGCCACCAUCSta | 73 |
| 14 | 14995 | 15015 | RNA-dependent RNA polymerase | GAGUUAUGAGGAUCAAGAU | 15 | AUCUUGAUCCUCAUAACUCat | 74 |
| 15 | 15002 | 15022 | RNA-dependent RNA polymerase | GAGGAUCAAGAUGCACUUU | 16 | AAAGUGCAUCUUGAUCCUCat | 75 |
| 16 | 15077 | 15097 | RNA-dependent RNA polymerase | GCCAUUAGUGCAAAGAAUA | 17 | UAUUCUUUGCACUAAUGGCat | 76 |
| 17 | 15079 | 15099 | RNA-deoendent RNA polymerase | CAUUAGUGCAAAGAAUAGA | 18 | UCUAUUCUUUGCACUAAUGgc | 77 |
| 18 | 15105 | 15125 | RNA-dependent RNA polymerase | CCGUAGCUGGUGUCUCUAU | 19 | AUAGAGACACCAGCUACGGtg | 78 |
| 19 | 15107 | 15127 | RNA-deoendent RNA polymerase | GUAGCUGGUGUCUCUAUCU | 20 | AGAUAGAGACACCAGCUACgg | 79 |
| 20 | 15110 | 15130 | RNA-dependent RNA polymerase | GCUGGUGUCUGUAUCUGUA | 21 | UACAGAUAGAGACACCAGCta | 80 |
| 21 | 15113 | 15133 | RNA-deoendent RNA polymerase | GGUGUCUCUAUCUGUAGUA | 22 | UACUACAGAUAGAGACACCag | 81 |
| 22 | 15116 | 15136 | RNA-deoendent RNA polymerase | GUCUCUAUCUGUAGUACUA | 23 | UAGUACUACAGAUAGAGACac | 82 |
| 23 | 15498 | 15518 | RNA-dependent RNA polymerase | CUGCUUAUGCUAAUAGUGU | 24 | ACACUAUUAGCAUAAGCAGtt | 83 |
| 24 | 15623 | 15643 | RNA-dependent RNA polymerase | GAGUGUCUCUAUAGAAAUA | 25 | UAUUUCUAUAGAGACACUCat | 84 |
| 25 | 15838 | 15858 | RNA-dependent RNA polymerase | GACUGAGACUGACCUUACU | 26 | AGUAAGGUCAGUCUCAGUCca | 85 |
| 26 | 15987 | 16007 | RNA-deoendent RNA polymerase | CAGAUGGUACACUUAUGAU | 27 | AUCAUAAGUGUACCAUCUGtt | 86 |
| 27 | 16061 | 16081 | RNA-denendent RNA polymerase | CAGGAGUAUGCUGAUGUCU | 28 | AGACAUCAGCAUACUCCUGat | 87 |
| 28 | 16063 | 16083 | RNA-deoendent RNA polymerase | GGAGUAUGCUGAUGUCUUU | 29 | AAAGACAUCAGCAUACUCCtg | 88 |
| 29 | 16193 | 16213 | RNA-dependent RNA polymerase | GAGUUUUAUGAGGCUAUGU | 30 | ACAUAGCCUCAUAAAACUCae | 89 |
| 30 | 16430 | 16450 | helicase | GGAGGUAUGAGCUAUUAUU | 31 | AAUAAUAGCUCAUACCUCCta | 90 |
| 31 | 16825 | 16845 | helicase | GUACACCUUUGAAAAAGGU | 32 | ACCUUUUUCAAAGGUGUACtc | 91 |
| 32 | 17022 | 17042 | helicase | CUAGCAAUGUUGCAAAUUA | 33 | UAAUUUGCAACAUUGCUAGaa | 92 |
| 33 | 17025 | 17045 | helicase | GCAAUGUUGCAAAUUAUCA | 34 | UGAUAAUUUGGAACAUUGCta | 93 |
| 34 | 17026 | 17046 | helicase | CAAUGUUGCAAAUUAUCAA | 35 | UUGAUAAUUUGCAACAUUGct | 94 |
| 35 | 17081 | 17101 | helicase | CCACCUGGUAGUGGUAAGA | 36 | UCUUACCAGUACCAGGUGGtc | 95 |
| 36 | 17255 | 17275 | helicase | GAGUGUUUUGAUAAAUUCA | 37 | UGAAUUUAUCAAAACACUCta | 96 |
| 37 | 17257 | 17277 | helicase | GUGUUUUGAUAAAUUCAAA | 38 | UUUGAAUUUAUCAAAACACtc | 97 |
| 38 | 17565 | 17585 | helicase | GUCCUGCUGAAAUUGUUGA | 39 | UCAACAAUUUCAGCAGGACaa | 98 |
| 39 | 17567 | 17587 | helicase | CCUGCUGAAAUUGUUGACA | 40 | UGUCAACAAUUUCAGCAGGac | 99 |
| 40 | 19570 | 19590 | 3'-to-5' exonuclease | CAAACAAUUUGAUACUUAU | 41 | AUAAGUAUCAAAUUGUUUGta | 100 |
| 41 | 21504 | 21524 | 2'-0-ribose methyltransferase | GAGAAAACAACAGAGUUGU | 42 | ACAACUCUGUUGUUUUCUCta | 101 |
| 42 | 22815 | 22835 | S | GCUGAUUAUAAUUAUAAAU | 43 | AUUUAUAAUUAUAAUCAGCaa | 102 |
| 43 | 22816 | 22836 | S | CUGAUUAUAAUUAUAAAUU | 44 | AAUUUAUAAUUAUAAUCAGca | 103 |
| 44 | 23956 | 23976 | S | GUUUUAAUUUUUCACAAAU | 45 | AUUUGUGAAAAAUUAAAACca | 104 |
| 45 | 24303 | 24323 | S | GUUCUCUAUGAGAACCAAA | 46 | UUUGGUUCUCAUAGAGAACat | 105 |
| 46 | 24624 | 24644 | S | GCUAAUCUUGCUGCUACUA | 47 | UAGUAGCAGCAAGAUUAGCag | 106 |
| 47 | 24625 | 24645 | S | CUAAUCUUSCUGCUACUAA | 48 | UUAGUAGCAGCAAGAUUAGca | 107 |
| 48 | 24630 | 24650 | S | CUUGCUGCUACUAAAAUGU | 49 | ACAUUUUAGUAGCAGCAAGat | 108 |
| 49 | 24664 | 24684 | S | GACAAUCAAAAAGAGUUGA | 50 | UCAACUCUUUUUGAUUGUCca | 109 |
| 50 | 25364 | 25384 | S | CAAAUUACAUUACACAUAA | 51 | UUAUGUGUAAUGUAAUUUGac | 110 |
| 51 | 26291 | 26311 | E | GUACUUCUUUUUCUUGCUU | 52 | AAGCAAGAAAAAGAAGUACgc | 111 |
| 52 | 26303 | 26323 | E | CUUGCUUUCGUGGUAUUCU | 53 | AGAAUACCACGAAAGCAAGaa | 112 |
| 53 | 26579 | 26599 | M | GGAACCUAGUAAUAGGUUU | 54 | AAACCUAUUACUAGGUUCCat | 113 |
| 54 | 26583 | 26603 | M | CCUAGUAAUAGGUUUCCUA | 55 | UAGGAAACCUAUUACUAGGtt | 114 |
| 55 | 26584 | 26604 | M | CUAGUAAUAGGUUUCCUAU | 56 | AUAGGAAACCUAUUACUAGgt | 115 |
| 56 | 28746 | 28766 | N | CUACAACUUCCUCAAGGAA | 57 | UUCCUUGAGGAAGUUGUAGca | 116 |
| 57 | 28749 | 28769 | N | CAACUUCCUCAAGGAACAA | 58 | UUGUUCCUUGAGGAAGUUGta | 117 |
| 58 | 28752 | 28772 | N | CUUCCUCAAGGAACAACAU | 59 | AUGUUGUUCCUUGAGGAAGtt | 118 |
| 59 | 28952 | 28972 | N | GAACCAGCUUGAGAGCAAA | 60 | UUUGCUCUCAAGCUGGUUCaa | 119 |

| | | | | | | | |
|---|---|---|---|---|---|---|---|
| *¹···Function of a nucleic acid comprising the target sequence or a protein encoded by the nucleic acid | | | | | | | |

293T cells (obtained from ACTT) were inoculated at 5.0 × 10⁴ cells/well to a 24-well plate. On the next day, a SARS-CoV-2 expression plasmid, a firefly luciferase (Flue) expression plasmid (control plasmid), and test substance siRNA or negative control siRNA were introduced into the cells using Lipofectamine 3000 Transfection Reagent (Thermo Fisher Scientific Inc.). The test substance siRNA was used at 30 or 100 nM, and Lipofectamine 3000 Transfection Reagent was used at 1.5 µL/well. The SARS-CoV-2 expression plasmid used was prepared by synthesizing as DNA artificial genes the genomic RNA of SARS-CoV-2 divided into ninth (Table 1-2), and subcloning these genes into expression plasmids pcDNA3.1. The artificial gene synthesis and the subcloning into expression plasmids were entrusted to Thermo Fisher Scientific Inc. However, since plasmid No. 4 could not be obtained, SARS-CoV-2 (2019-nCoV) 3C-like proteinase/3CLpro Gene ORF cDNA clone expression plasmid (Sino Biological Inc.) was used in the activity evaluation of some siRNAs. The control plasmid used was pGL4.50 [luc2/CMV/Hygro] Vector (Promega Inc.). The synthesis of the test substance siRNA was entrusted to Japan Bio Service Co. The negative control siRNA used was MISSION siRNA Universal Negative Control (Sigma-Aldrich Co., LLC).

Two days after introduction of each siRNA and the corresponding plasmid, RNA was isolated from the cells using NucleoSpin RNA (Macherey-Nagel GmbH & Co. KG) and subjected to reverse-transcription (RT) reaction using High Capacity cDNA Reverse Transcription Kit with RNase Inhibitor (Thermo Fisher Scientific Inc.) and random primers attached to the kit. qPCR was performed with the RT reaction solution as a template using Fast SYBR Green Master Mix (Thermo Fisher Scientific Inc.) according to manufacturer's protocol to measure an expression level of SARS-CoV-2 RNA derived from the SARS-CoV-2 expression plasmid. Table 1-2 shows the primers used in the detection of SARS-CoV-2 RNA derived from the SARS-CoV-2 expression plasmid. The SARS-CoV-2 RNA expression level was corrected with an expression level of Flue RNA derived from the control plasmid or a PPIB RNA expression level of a housekeeping gene. Table 1-3 shows the primers used in the detection of Fluc RNA or PPIB RNA. The ratio of the SARS-CoV-2 RNA expression level resulting from the introduction of the test substance siRNA to the SARS-CoV-2 RNA expression level resulting from the introduction of the negative control siRNA was calculated and analyzed for the knockdown activity of the test substance siRNA. The results are shown in Table 1-4.

**[Table 1-2]**

| Table 1-2 SARS-CoV-2 genome subcloned into expression plasmid, and primer for detection | | | | | | | | | | | |
|---|---|---|---|---|---|---|---|---|---|---|---|
| Plasmid No. | Position in SEQ ID NO: 1 | | Strand length | Primer set for detection | | | | Primer set 2 for detection | | | |
| | Start | End | | Sense primer 5' to 3' | SEQ ID NO | Reverse primer 5' to 3' | SEQ ID NO | Sense primer 5 to 3' | SEQ ID NO | Reverse trimer 5' to 3' | SEQ ID NO |
| 1 | 1 | 2779 | 2779 | CGTACGTGGCTTTGGAGACT | 120 | TCGAGOATCCGAACGTTTGA | 121 | TCATGCCTCTAAAAGCCCCA | 122 | TCGAGCAACATAAGCCGTT | 123 |
| 2 | 2660 | 8260 | 3601 | ACCTTTBGAATTBBTGCCAC | 124 | TTGTCCTCACTGCCGTCTTB | 125 | GGACAACABCAGACAACCCT | 126 | AGTTCATACTGAGCAGCGAGBTGGTG | 127 |
| 3 | 6141 | 10114 | 3974 | GAAGAGGTTGGCCACACAGA | 128 | AGCAGCTAAACCATGAGTABCA | 129 | TTTGAAGAAGCTBCBCTGTG | 130 | GCGAGATGACAACAAGCAGC | 131 |
| 4 | 9995 | 13602 | 36C8 | - | | - | | - | | - | |
| 3CLpro | 10055 | 10972 | 918 | CCCATCTGGTAAAGTTGAGGGT | 132 | AGCATGTCTTCAGAGGTGCA | 133 | TBCTCAAACTBBAATTBCCB | 134 | TTGGAAAGTAACACCTGAGCA | 135 |
| 6 | 133B2 | 18099 | 4718 | AAGGATTGTCCAGCTGTTGC | 136 | AGACGAGGTCTGCCATTGTB | 137 | ACGTAACCCTGCTTGGAGAAA | 138 | TGAGCCCTGTBATGAATCAACA | 139 |
| 6 | 179B0 | 21613 | 3634 | TGTACGTGCATGGATTGGCT | 140 | CAGGCGGTGGTTTAGCACTA | 141 | TCTTGGAGGTTCCBTBGCTA | 142 | AGTAACAAAGGCTBTCCACCA | 143 |
| 7 | 21502 | 25443 | 3942 | CATOTCTCTGGGACCCAATGGT | 144 | AGTAGGGACTBBBTCTTCBA | 115 | TGTCCTTCCCTCABTCAGCA | 146 | TGACAAATGGCAGGAGCAGT | 147 |
| 8 | 25334 | 26522 | 1189 | CGGATOGCTTATTQTTGGCQ | 148 | TGAACACCCTTGGABAGTGC | 149 | TGABTACAGACACTGGTGTTGA | 150 | GGATGAACCGTCGATTGTGTG | 151 |
| 9 | 26473 | 29970 | 3398 | GGCTTGATQTGGCTCAGCTA | 152 | GBABTBBCACBTTBABAAGA | 153 | AGACCACACAAGGCABATGG | 154 | TCGGTGAAAATGTGGTGGCT | 155 |

**[Table 1-3]**

| Table 1-3 Gene and primer for detection used in correction of SARS-CoV-2 expression level | | | | |
|---|---|---|---|---|
| Gene for correction | Primer set for detection | | | |
| | Sense primer 5' to 3' | SEQ ID NO | Reverse primer 5' to 3' | SEQ ID NO |
| Fluc | AACCAGCGCCATTCTGATCA | 156 | TCGGGGTTGTTAACGTAGCC | 157 |
| PPIB | CAGCAAATTCCATCGTG | 158 | CCGTAGTGCTTCAGTTT | 159 |

**[Table 1-4]**

| Table 1-4 Knockdown activity of siRNA using SARS-CoV-2 expression plasmid | | | | |
|---|---|---|---|---|
| siRNA No. | Plasmid No. | Function of target sequence (*1) | KD activity measurement using plasmid | |
| | | | SARS-CoV-2 RNA knockdown rate | |
| | | | Flue correction | PPIB correction |
| 1 | 1 | 5'UTR | 44% | 15% |
| 2 | | | 56% | 32% |
| 3 | 2 | nsp3 | 63% | 31% |
| 4 | 3 | | 44% | 33% |
| 7 | 3CLpro | 3C-like proteinase | 40% | 23% |
| 10 | 5 | RNA-dependent RNA polymerase | 35% | 71% |
| 11 | | | 49% | 51% |
| 12 | | | 63% | 40% |
| 13 | | | 41% | 35% |
| 14 | | | 51% | 55% |
| 15 | | | 59% | 71% |
| 16 | | | 67% | 57% |
| 17 | | | 64% | 46% |
| 18 | | | 57% | 44% |
| 27 | | | 48% | 30% |
| 28 | | | 50% | 63% |
| 33 | | helicase | 57% | 18% |
| 34 | | | 44% | 41% |
| 35 | | | 39% | 45% |
| 36 | | | 48% | 48% |
| 38 | | | 58% | 47% |
| 39 | | | 53% | 54% |
| 41 | 6 | 2'-O-ribose methyltransferase | 35% | 22% |
| 48 | 7 | S | 56% | 52% |
| 54 | 9 | M | 42% | 30% |
| 57 | | N | 76% | 72% |
| 58 | | | 48% | 40% |
| 59 | | | 48% | 16% |

| | | | | |
|---|---|---|---|---|
| *1···Function of a nucleic acid comprising the target sequence or a protein encoded by the nucleic acid | | | | |

### <Example 2: Measurement of knockdown activity of siRNA using SARS-CoV-2 virus (1st screening)>

A mixture of 30 or 100 nM (final concentration) of test substance siRNA or negative control siRNA and Lipofectamine 3000 Transfection Reagent (Thermo Fisher Scientific Inc., 0.3 µL/well) was added to a 96-well plate, to which cells stably expressing human ACE2 (293T-ACE2) prepared by the infection of 293T cells with a human ACE2 expression lentivirus vector were inoculated at 3.0 × 10⁴ cells/well. The negative control siRNA used was MISSION siRNA Universal Negative Control (Sigma-Aldrich Co., LLC). On the next day, a SARS-CoV-2 virus solution was added thereto at MOI = 0.01 so that the cells were infected with the virus. The SARS-CoV-2 virus used was recombinant SARS-CoV-2 virus (rSARS-CoV-2-ORF8-Nluc) in which nano-luciferase gene was incorporated in a coding region of an accessory protein ORF8 using the same approach as that described in the literature (Terada et al. 2019. J Virol 93: e01208-19. https://doi.org/10.1128/JVI.01208-19) on the basis of a WK521 strain obtained from National Institute of Infectious Diseases, Japan. One hour after virus solution addition, the virus in the medium was removed by medium replacement. Twenty-four hours after viral infection, the culture supernatant was collected, and the cells were prepared into a cell lysate using Passive Lysis Buffer (Promega Inc.). Luciferase activity in the cell lysate was measured using Luciferase Assay System (Promega Inc.) according to the protocol attached thereto to evaluate the amount of the SARS-CoV-2 virus in the cells. Also, 5 µL of the collected culture supernatant was added to a 96-well plate separately inoculated with 293T-ACE2 cells at 3.0 × 10⁴ cells/well so that the cells were infected with the virus in the culture supernatant. Twenty-four hours after infection with the culture supernatant, the amount of the SARS-CoV-2 virus in the cells was evaluated in the same manner as in the first viral infection to evaluate the amount of infectious viral particles in the culture supernatant. Further, RNA was isolated from the collected culture supernatant using QIAamp Viral RNA Mini Kit (Qiagen) and subjected to one-step qPCR for quantifying SARS-CoV-2 virus RNA using THUNDERBIRD Probe One-step qRT-PCR Kit (Toyobo Co., Ltd.). The primers and the probe used were N Set No. 2 (sense primer 5'-AAATTTTGGGGACCAGGAAC-3' (SEQ ID NO: 160), reverse primer 5'-TGGCAGCTGTGTAGGTCAAC-3' (SEQ ID NO: 161), and probe 5'-FAM-ATGTCGCGCATTGGCATGGA-TAMRA-3' (SEQ ID NO: 162)) described in "Pathogen Detection Manual 2019-nCoV Ver. 2.9.1" (https://www.niid.go.jp/niid/ja/labo-manual.html#class0) of National Institute of Infectious Diseases, Japan. The results are shown in Table 2.

**[Table 2]**

| Table 2 measurement of knockdown activity of siRNA using SARS-CoV-2 virus (1st screening) | | | | | | |
|---|---|---|---|---|---|---|
| siRNA No. | Function of target sequence (*1) | measurement of KD activity using virus (1st screening) | | | | |
| | | Suppression rate of amount of intracellular virus | | Suppression rate of amount of infectious viral particle | | Suppression rate of qPCR of supernatant |
| | | 30nM | 100nM | 30nM | lOOnM | 30nM |
| 1 | 5'UTR | 92% | 93% | 95% | 93% | |
| 2 | | 95% | 98% | 99% | 99% | |
| 3 | nsp3 | 61% | 70% | 66% | 70% | |
| 4 | | 76% | 75% | 48% | 63% | |
| 7 | 3C-like proteinase | 34% | 42% | 34% | 48% | - |
| a | nsp9 | 43% | 48% | 61% | 63% | |
| 11 | RNA-dependent RNA polymerase | 35% | 41% | 44% | 42% | 70% |
| 12 | | 3% | 6% | 59% | 43% | 85% |
| 14 | | 45% | 43% | 57% | 53% | 83% |
| 16 | | 46% | 61% | 63% | 72% | 1% |
| 17 | | 43% | 57% | 54% | 55% | 69% |
| 28 | | 94% | 96% | 97% | 98% | 97% |
| 33 | helicase | 88% | 92% | 96% | 96% | 74% |
| 34 | | 82% | 80% | 92% | 92% | 94% |
| 35 | | 61% | 67% | 91% | 93% | -1% |
| 36 | | 64% | 71% | 79% | //% | |
| 38 | | 54% | 60% | 63% | 10% | |
| 39 | | 48% | 51% | 69% | 75% | |
| 41 | 2'-O-ribose methyltransferase | 50% | 51% | 63% | 60% | - |
| 57 | N | 93% | 93% | 99% | 99% | 94% |
| 58 | | 79% | 85% | 90% | 89% | |
| 59 | | 96% | 9/% | 99% | 99% | |

| | | | | | | |
|---|---|---|---|---|---|---|
| ^{*1···}Function of a nucleic acid comprising the target sequence or a protein encoded by the nucleic acid | | | | | | |

### <Example 3: Measurement knockdown activity of siRNA using SARS-CoV-2 virus (2nd screening)>

In Example 2, viral infection was performed after siRNA introduction. In this Example, siRNA was introduced after viral infection.

Specifically, 293T-ACE2 cells were inoculated at 3.0 × 10⁴ cells/well to a 96-well plate. On the next day, a SARS-CoV-2 virus solution was added thereto at MOI = 0.01 so that the cells were infected with the virus. The SARS-CoV-2 virus used was the same SARS-CoV-2 virus (rSARS-CoV-2-ORF8-Nluc) as in Example 2. One hour after viral solution addition, the virus in the medium was removed by medium replacement. siRNA was introduced to the cells by adding thereto a mixture of test substance siRNA or negative control siRNA and Lipofectamine 3000 Transfection Reagent (Thermo Fisher Scientific Inc., 0.1 µL/well). The final concentration of the siRNA was 3 or 10 nM. Twenty-four hours or 48 hours after viral infection, the culture supernatant was collected, and the cells were prepared into a cell lysate using Passive Lysis Buffer (Promega Inc.). Luciferase activity in the cell lysate was measured using Luciferase Assay System (Promega Inc.) according to the protocol attached thereto to evaluate the amount of the SARS-CoV-2 virus in the cells. Also, 5 µL of the collected culture supernatant was added to a 96-well plate separately inoculated with 293T-ACE2 cells at 3.0 × 10⁴ cells/well so that the cells were infected with the virus in the culture supernatant. Twenty-four hours after infection with the culture supernatant, the amount of the SARS-CoV-2 virus in the cells was evaluated in the same manner as in the first viral infection to evaluate the amount of infectious viral particles in the culture supernatant. The results are shown in Table 3.

**[Table 3]**

| Table 3 Measurement of knockdown activity of siRNA using SARS-CoV-2 virus (2nd screening) | | | | | | | | | |
|---|---|---|---|---|---|---|---|---|---|
| siRNA No. | Function of target sequence (*1) | KD activity measurement using virus (2nd screening) | | | | | | | |
| | | Suppression rate of amount of intracellular virus | | | | Suppression rate of amount of infectious viral particle | | | |
| | | 24h | | 48h | | 24h | | 48h | |
| | | 3nM | 10nM | 3nM | 10nM | 3nM | 10nM | 3nM | 10nM |
| 2 | 5'UTR | 25% | 24% | 25% | 29% | -6% | 31% | 82% | 84% |
| 34 | helicase | 33% | 22% | 10% | 20% | 37% | 22% | 7% | 76% |
| 35 | | 30% | 22% | 15% | 14% | 51% | 42% | 75% | 69% |
| 57 | N | 31% | 35% | 16% | 17% | 46% | 48% | 69% | 83% |
| 58 | | 41% | 22% | 19% | 10% | 8% | 45% | 64% | 43% |

| | | | | | | | | | |
|---|---|---|---|---|---|---|---|---|---|
| _{*}1···Function of a nucleic acid comprising the target sequence or a protein encoded by the nucleic acid | | | | | | | | | |

## Claims

1. An antiviral nucleic acid or a pharmaceutically acceptable salt or hydrate thereof targeting a sequence in at least one target region selected from the group consisting of 5' UTR region, nsp3 region, 3C-like proteinase region, nsp9 region, RNA-dependent RNA polymerase region, helicase region, 3'-to-5' exonuclease region, 2'-O-ribose methyltransferase region, S region including S1 region and S2 region, E region, M region, and N region in genomic RNA of SARS-CoV-2, wherein
the virus is SARS-CoV-2, SARS-CoV-1, or MERS-CoV.

2. The nucleic acid or the pharmaceutically acceptable salt or hydrate thereof according to claim 1, wherein the target sequence is a sequence of 16 to 30 consecutive bases in the target region.

3. The nucleic acid or the pharmaceutically acceptable salt or hydrate thereof according to claim 1 or 2, wherein the virus is SARS-CoV-2 or SARS-CoV-1.

4. The nucleic acid or the pharmaceutically acceptable salt or hydrate thereof according to claim 3, wherein the nucleic acid or the pharmaceutically acceptable salt or hydrate thereof targets a sequence in at least one target region selected from the group consisting of positions 43 to 69, positions 242 to 279, positions 3352 to 3372, positions 6406 to 6427, positions 10406 to 10431, positions 10484 to 10506, positions 12839 to 12867, positions 12898 to 12922, positions 12965 to 12990, positions 13481 to 13502, positions 13762 to 13790, positions 13894 to 13916, positions 14290 to 14312, positions 14654 to 14687, positions 14750 to 14777, positions 14824 to 14846, positions 14878 to 14909, positions 14953 to 14990, positions 14992 to 15026, positions 15037 to 15061, positions 15063 to 15140, positions 15172 to 15198, positions 15278 to 15299, positions 15454 to 15479, positions 15496 to 15518, positions 15622 to 15644, positions 15829 to 15859, positions 15929 to 15950, positions 15985 to 16011, positions 16051 to 16085, positions 16189 to 16220, positions 16430 to 16451, positions 16822 to 16845, positions 17015 to 17051, positions 17080 to 17103, positions 17254 to 17277, positions 17564 to 17600, positions 18196 to 18218, positions 18253 to 18278, positions 19568 to 19595, positions 20888 to 20909, positions 21502 to 21524, positions 22814 to 22836, positions 23093 to 23113, positions 23956 to 23976, positions 24302 to 24324, positions 24467 to 24489, positions 24620 to 24651, positions 24662 to 24684, positions 24962 to 24982, positions 25104 to 25128, positions 25364 to 25384, positions 26287 to 26325, positions 26574 to 26604, positions 28274 to 28294, positions 28397 to 28418, positions 28509 to 28538, positions 28744 to 28784, positions 28799 to 28820, positions 28946 to 28972, positions 29010 to 29031, positions 29102 to 29130, and positions 29174 to 29196 of SEQ ID NO: 1.

5. The nucleic acid or the pharmaceutically acceptable salt or hydrate thereof according to claim 1, wherein the nucleic acid or the pharmaceutically acceptable salt or hydrate thereof targets a sequence in at least one target region selected from the group consisting of 5' UTR region, helicase region, and N region in genomic RNA of SARS-CoV-2.

6. The nucleic acid or the pharmaceutically acceptable salt or hydrate thereof according to any one of claims 1 to 5, wherein the nucleic acid is siRNA or shRNA.

7. The nucleic acid or the pharmaceutically acceptable salt or hydrate thereof according to claim 6, comprising
a sense strand comprising
(a) a base sequence selected from the group consisting of SEQ ID NOs: 2 to 60,
(b) a base sequence having addition, deletion, or substitution of one or several base(s) in the base sequence selected from the group consisting of SEQ ID NOs: 2 to 60, or
(c) a base sequence having at least 80% sequence identity to the base sequence selected from the group consisting of SEQ ID NOs: 2 to 60, and
an antisense strand comprising a base sequence complementary to any of the sequences (a) to (c),
wherein the nucleic acid or the pharmaceutically acceptable salt or hydrate thereof inhibits a function of the target region.

8. The nucleic acid or the pharmaceutically acceptable salt or hydrate thereof according to claim 7, comprising
a sense strand comprising
(a) a base sequence selected from the group consisting of SEQ ID NOs: 2, 3, 4, 5, 8, 9, 11 to 19, 28, 29, 34 to 37, 39, 40, 42, 49, 55, and 58 to 60,
(b) a base sequence having addition, deletion, or substitution of one or several base(s) in the base sequence selected from the group consisting of SEQ ID NOs: 2, 3, 4, 5, 8, 9, 11 to 19, 28, 29, 34 to 37, 39, 40, 42, 49, 55, and 58 to 60, or
(c) a base sequence having at least 80% sequence identity to the base sequence selected from the group consisting of SEQ ID NOs: 2, 3, 4, 5, 8, 9, 11 to 19, 28, 29, 34 to 37, 39, 40, 42, 49, 55, and 58 to 60, and
an antisense strand comprising a base sequence complementary to any of the sequences (a) to (c),
wherein the nucleic acid or the pharmaceutically acceptable salt or hydrate thereof inhibits a function of the target region.

9. The nucleic acid or the pharmaceutically acceptable salt or hydrate thereof according to claim 8, comprising
a sense strand comprising
(a) a base sequence selected from the group consisting of SEQ ID NOs: 3, 35, 36, 58, and 59,
(b) a base sequence having addition, deletion, or substitution of one or several base(s) in the base sequence selected from the group consisting of SEQ ID NOs: 3, 35, 36, 58, and 59, or
(c) a base sequence having at least 80% sequence identity to the base sequence selected from the group consisting of SEQ ID NOs: 3, 35, 36, 58, and 59, and
an antisense strand comprising a base sequence complementary to any of the sequences (a) to (c),
wherein the nucleic acid or the pharmaceutically acceptable salt or hydrate thereof inhibits a function of the target region.

10. The nucleic acid or the pharmaceutically acceptable salt or hydrate thereof according to any one of claims 1 to 9, wherein the sugar moiety and/or the phosphate bond moiety of at least one nucleotide constituting the nucleic acid are/is modified.

11. The nucleic acid or the pharmaceutically acceptable salt or hydrate thereof according to any one of claims 1 to 10, wherein
the sugar moiety of at least one nucleotide constituting the nucleic acid is a ribose, and the 2'-OH group of the ribose is replaced by F or OCH₃, and/or
the nucleic acid is siRNA having an overhang, the sugar moiety of a nucleotide of the overhang moiety comprises a deoxyribose, and the 2'-H group of the deoxyribose is optionally replaced by F or OCH₃.

12. A vector comprising the nucleic acid according to any one of claims 1 to 11 or DNA encoding the nucleic acid.

13. A pharmaceutical composition comprising the nucleic acid or the pharmaceutically acceptable salt or hydrate thereof according to any one of claims 1 to 11 or the vector according to claim 12.

14. The pharmaceutical composition according to claim 13 for the treatment and/or prevention of viral infection, wherein the virus is SARS-CoV-2, SARS-CoV-1, or MERS-CoV.

15. A method for treating and/or preventing viral infection, comprising administering to a subject an effective amount of the nucleic acid or the pharmaceutically acceptable salt or hydrate thereof according to any one of claims 1 to 11, the vector according to claim 12, or the pharmaceutical composition according to claim 13 or 14, wherein the virus is SARS-CoV-2, SARS-CoV-1, or MERS-CoV.
